(19) Europäisches Patentamt
European Patent Office
Office européen des brevets

(11) **EP 4 286 846 A1**

(12) **EUROPEAN PATENT APPLICATION**


(43) Date of publication:
**06.12.2023 Bulletin 2023/49**

(21) Application number: **22177321.1**

(22) Date of filing: **03.06.2022**

(51) International Patent Classification (IPC):
***G01N 33/50*** *(2006.01)* ***G01N 33/58*** *(2006.01)*

(52) Cooperative Patent Classification (CPC):
**G01N 33/502; G01N 33/582**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **Max-Planck-Gesellschaft zur Förderung der Wissenschaften e.V.**
**80539 München (DE)**

(72) Inventors:
- **Fritsch, Anatol W.**
**01097 Dresden (DE)**
- **Artola, Juan Manuel Iglesias**
**01097 Dresden (DE)**
- **Hyman, Anthony A.**
**01309 Dresden (DE)**

(74) Representative: **Arth, Hans-Lothar**
**ABK Patent Attorneys**
**Jasminweg 9**
**14052 Berlin (DE)**


Remarks:
The complete document including Reference Table(s) and the Sequence Listing(s) can be downloaded from the EPO website

# (54) ACCURATE METHOD FOR GENERATING A PHASE DIAGRAM OF A POLYMER


(57) The present invention refers to a method for generating a binodal curve of a polymer in a system by determining accurate values of both concentration of dilute and condensed phases of a polymer, in particular, protein or polynucleic acid, under different condition, such as at different temperatures and salt concentrations.

Furthermore, the present invention refers to an assay method for identify bioactive compound(s), comprising the inventive method for generating a binodal curve of a polymer in a system.


Figure 10

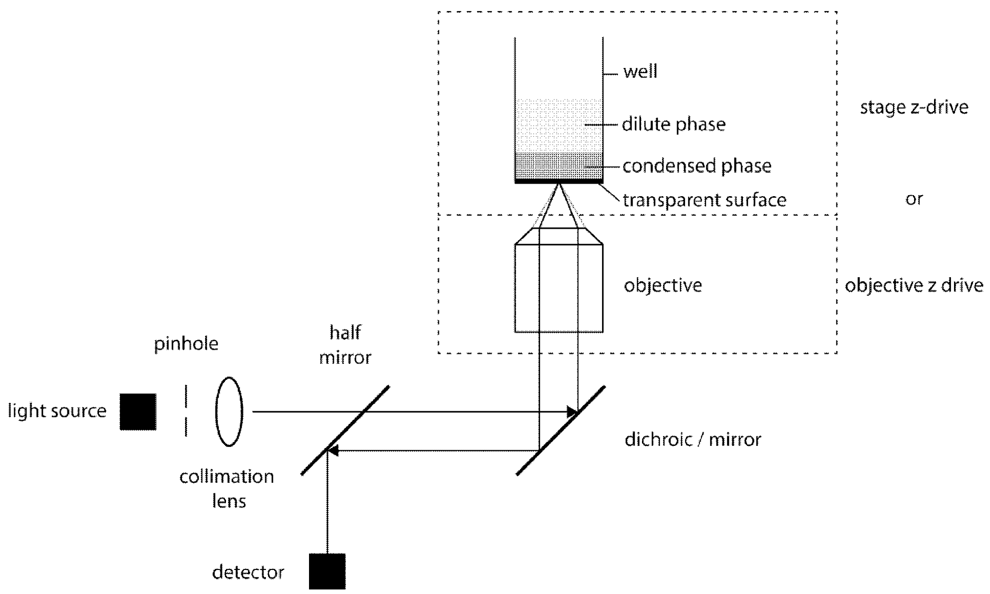


EP 4 286 846 A1

## Description

### Specification

The present invention refers to a method for generating a binodal curve of a polymer in a system by determining accurate concentration values for both the dilute and condensed phases of a polymer, in particular, protein or polynucleic acid, under different conditions, such as at different temperatures, pH, salt concentrations, pressure, crowding agents, buffer compositions. The method can be applied to in vitro and in vivo systems alike.

Furthermore, the present invention refers to an assay method for identifying bioactive compound(s), comprising the inventive method for generating a binodal curve of a polymer in a system.

### Background of the invention

Protein phase separation has become a widely studied phenomenon in biology with implications in cell metabolism and disease. An accurate study of phase separating proteins relies on the precise determination of the binodal curves. The binodal curves in these phase diagrams give information on the protein concentration required for condensate formation and the respective concentration inside the condensate at defined external conditions (temperature, salt, pH, pressure, buffer composition, crowding agent). However, it has proven difficult to measure accurate binodal curves and, in general, phase diagrams.

Protein condensates are present in a wide variety of cells where they perform fundamental functions in development (e. g. P granules in *C. elegans*), ageing (e.g. stress granules) and in diverse cellular processes (e.g. transcription). They are known to form through a thermodynamic process, referred to as phase separation, that results from favourable interactions between proteins and between proteins and polynucleic acids. The strength and nature of these intermolecular interactions are governed by the protein sequence and secondary structure, and by the cellular environment. Changes in sequence and environment can therefore dictate the protein concentrations necessary to achieve phase separation and the resulting material properties of the condensates. Temperature, salt concentration and pH are important external control parameters of thermodynamic phase separation.

Typically, high salt concentrations lead to condensate dissolution by shielding charge interactions between and within proteins. A similar effect is observed with temperature, where the solvation state and the secondary structure of the protein are affected. From these considerations, it follows that to understand the phase separation of a given protein, its phase diagrams must be constructed in respect to the relevant variables under study. The complete phase diagram maps the control parameter(s) (temperature, salt concentrations, etc.) with the dilute and the condensed phases of a system via a binodal curve.

Thus far it has proven challenging to produce accurate phase diagrams for a wide variety of proteins as a consequence of sample constrains and technical requirements. Ideally, it should be possible to determine binodal curves using a small volume of protein sample and readily accessible laboratory equipment. While dilute phase protein concentrations, or saturation concentrations, have been reported in numerous studies; the condensed phase concentration is more complicated to be measured and usually requires large amounts of protein (Brady, J. P. et al. Structural and hydrodynamic properties of an intrinsically disordered region of a germ cell-specific protein on phase separation. PNAS, 2017, 114, E8194-E8203; McCall, P. M. et al. Quantitative phase microscopy enables precise and efficient determination of biomolecular condensate composition. 2020.10.25.352823; Bracha, D. et al. Mapping Local and Global Liquid Phase Behavior in Living Cells Using Photo-Oligomerizable Seeds. Cell, 2018, 175, p.1467-1480.).

This is in part because of the difficulties that arise from quantitative fluorescence-based approaches as a consequence of fluorophore quenching caused by changes in the environment inside the condensate and optical limitations. Resourcing to bulk approaches is often not accessible due to the amount of protein that would be required for measurement.

In this work we present a simple and accurate approach that is based on mass and volume conservation and defined reaction volumes to determine binodal curves for phase separating polymers.

It is the objective of the present invention to provide a method for determining accurate concentration values for both the dilute and condensed phases of a polymer, in particular, protein or polynucleic acid, under different conditions, such as at different temperatures and salt concentrations in a system for generating binodal curves for a phase diagram of said polymer.

The present invention can be applied to *in vitro* and *in vivo* systems alike.

Furthermore, the present invention refers to an assay method for identify bioactive compound(s), comprising the inventive method for generating a binodal curve of a polymer in a system.

In this invention, a simple and accurate method for producing binodal curves for phase diagrams is provided by determining both dilute and condensed phase concentrations of polymer mixtures. The method of the present invention overcomes the above-mentioned difficulties. It builds upon an accurate determination of the condensed phase volume

fraction in an environment of known volume and a titration of polymer concentration. It allows determining accurate phase diagrams in a rapid manner.

The objective of the present invention is solved by the teaching of the independent claims. Further advantageous features, aspects and details of the invention are evident from the dependent claims, the description, the figures, and the examples of the present application.

**Brief Summary of the Invention**

The present invention refers to a method for determining a concentration ($c_{con}$) of a polymer in a condensed phase and a concentration ($c_{dil}$) of the polymer in a dilute phase in a system comprising:

A) determining a total volume ($V_{tot}$) of the system and a total concentration ($c_{tot}$) of the polymer;
B) triggering phase separation of the polymer into a condensed phase and a dilute phase in said system;
C) determining a volume fraction ($V_{con}$/ $V_{tot}$) of a condensed phase of the polymer in said system;
D) determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con} - c_{dil}} \cdot c_{tot} - \frac{c_{dil}}{c_{con} - c_{dil}},$$

E) changing at least one condition of said system and repeating the steps A) to D).

Preferably, the polymer is a protein, or a polynucleic acid, preferably RNA, DNA, or a mixture of a protein and RNA, or a mixture of a protein and DNA.

The inventors have derived a linear relationship between the volume fraction ($V_{con}$ / $V_{tot}$) of the condensed phase in a closed system and the total concentration of the polymer. Therefore, in a closed system, in a demixed state having two coexisting phases, a dilute and a condensed phase, the concentration of a polymer in both phases can be determined accurately from the volume fraction ($V_{con}$ / $V_{tot}$) of the polymer in the condensed phase at different total polymer concentrations $c_{tot}$. The condensed phase concentration is usually complicated to be measured and requires large amounts of protein when being directly measured (see Brady, J. P. et al. Structural and hydrodynamic properties of an intrinsically disordered region of a germ cell-specific protein on phase separation. PNAS, 2017, 114, E8194-E8203). With the inventive methods described herein, the polymer concentration in both phases can be determined accurately from easily obtainable parameters in a single experiment.

In other words, the present invention refers to a method for simultaneously determining a concentration ($c_{con}$) of a polymer in a condensed phase and a concentration ($c_{dil}$) of the polymer in a dilute phase in a system comprising:

A) determining a total volume ($V_{tot}$) of the system and a total concentration ($c_{tot}$) of the polymer;
B) triggering phase separation of the polymer into a condensed phase and a dilute phase in said system;
C) determining a volume fraction ($V_{con}$/ $V_{tot}$) of a condensed phase of the polymer in said system;
D) determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con} - c_{dil}} \cdot c_{tot} - \frac{c_{dil}}{c_{con} - c_{dil}},$$

E) changing at least one condition of said system and repeating the steps A) to D).

Reworded, the present invention refers to a method for determining a concentration ($c_{con}$) of a polymer in a condensed phase and a concentration ($c_{dil}$) of the polymer in a dilute phase in a system comprising:

A) providing specimens of a system comprising the polymer, each specimen having a total volume $V_{tot}$ and a different total concentration ($c_{tot}$) of the polymer;
B) triggering phase separation of the polymer into a condensed phase and a dilute phase in each specimen;
C) determining a volume fraction ($V_{con}$/ $V_{tot}$) of a condensed phase of the polymer in each specimen;
D)determining a concentration ($c_{con}$) of the condensed phase of the polymer and a concentration ($c_{dil}$) of the dilute phase of the polymer in each specimen by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) changing at least one condition of said system and repeating the steps A) to D).

Determining the polymer concentration in both phases in a single experiment also allows accurate binodal curve determination and thus phase diagram construction in a rapid manner. Conventional methods for binodal curve generation of a polymer are based on the determination of the concentration of the polymer in a dilute phase only and allow therefore only the determination of one side of the coexistence curve (binodal) in a single experiment. Thus, for the generation of a complete binodal curve and a complete phase diagram multiple sample preparations and experiments are required in order to obtain the concentration in the condensed phase.

In other words, the present invention refers to a method for determining a concentration ($c_{con}$) of a polymer in a condensed phase and a concentration ($c_{dil}$) of the polymer in a dilute phase in a system for generating a binodal curve comprising:

A) determining a total volume ($V_{tot}$) of the system and a total concentration ($c_{tot}$) of the polymer;
B) triggering phase separation of the polymer into a condensed phase and a dilute phase in said system;
C) determining a volume fraction ($V_{con}$/ $V_{tot}$) of a condensed phase of the polymer in said system;
D) determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) changing at least one condition of said system and repeating the steps A) to D).

Also, the present invention refers to a method for generating a binodal curve of a polymer in a system comprising:

A) determining a total volume ($V_{tot}$) of the system and a total concentration ($c_{tot}$) of the polymer;
B) triggering phase separation of the polymer into a condensed phase and a dilute phase in said system;
C) determining a volume fraction ($V_{con}$/ $V_{tot}$) of a condensed phase of the polymer in said system;
D)determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) changing at least one condition of said system and repeating the steps A) to D).

Preferably, in step E) the at least one condition of said system is selected from a concentration of a component in the system wherein the component is selected from a salt, a crowding agent, or a buffer; the pH value; the pressure; or the temperature of the system, or a combination of the aforementioned conditions.

Preferably, in the method of the invention, the system is an *in vitro* system and selected from a solution, an emulsion, or cells.

Preferably, in the step B) of the method of present invention, the phase separation of the polymer in said system is triggered by changing: the concentration of a component in the system selected from a salt, a crowding agent, or a buffer; the pH value; the pressure; or the temperature of the system. Preferably, the salt is selected from potassium chloride, sodium chloride, magnesium chloride, or any other solute.

Preferably, in the method of the invention, when the polymer is not labelled with a fluorophore or a fluorescent protein, in step A)

the total volume ($V_{tot}$) of said system and the total concentration ($c_{tot}$) of the polymer are determined by means of bright-field, dark-field, phase-contrast, holographic, polarization, or differential interference correlation (DIC) microscopy, or light-scattering based approaches.

Preferably, in the method of the invention, when the polymer is labelled with a fluorophore or a fluorescent protein, in the step A)

the total volume ($V_{tot}$) of said system and the total concentration ($c_{tot}$) of the polymer are determined by means of fluorescence microscopy.

In some aspects, in step C) of the method of the present invention, step C) comprises:

C1a) encapsulating a portion of the system in an emulsion system immediately;
C2a) determining a volume ($V_{em}$) of one emulsion droplet of the emulsion system, and a condensed volume ($V_{con}$') of the condensed phase of the polymer in said emulsion system;
C3a) determining a volume fraction ($V_{con'}/V_{em}$) of the condensed volume ($V_{con}$') of the condensed phase of the polymer in said emulsion system and the volume ($V_{em}$) of one emulsion droplet of said emulsion system, wherein the measured volume fraction ($V_{con'}/V_{em}$) is identical to the volume fraction ($V_{con}/V_{tot}$) of the condensed volume ($V_{con}$) of the condensed phase of the polymer in the system and the total volume ($V_{tot}$) of the system.

The emulsion system can be a water-in-oil emulsion system, an oil-in-water emulsion system or an oil-in-oil emulsion system. Preferably, the emulsion system is a water-in-oil emulsion system.

Thus, in a preferred embodiment of the method of the present invention, step C) comprises:

C1a) encapsulating a portion of the system in a water-in-oil emulsion system immediately;
C2a) determining a volume ($V_{em}$) of one emulsion droplet of the water-in-oil emulsion system, and a condensed volume ($V_{con}$') of the condensed phase of the polymer in said water-in-oil emulsion system;
C3a) determining a volume fraction ($V_{con'}/V_{em}$) of the condensed volume ($V_{con}$') of the condensed phase of the polymer in said water-in-oil emulsion system and the volume ($V_{em}$) of one emulsion droplet of said water-in-oil emulsion system, wherein the measured volume fraction ($V_{con'}/V_{em}$) is identical to the volume fraction ($V_{con}/V_{tot}$) of the condensed volume ($V_{con}$) of the condensed phase of the polymer in the system and the total volume ($V_{tot}$) of the system.

Preferably, when the polymer is not labelled with a fluorophore or a fluorescent protein, further comprising before step C1a):
C1a') adding fluorescent polymer beads and/or a fluorescent protein and/or a fluorescent dye partitioning into one of the phases of the system to label the condensed phase of the polymer.

Preferably, in the step C2a), a volume ($V_{em}$) of the emulsion system, and a condensed volume ($V_{con}$') of the condensed phase of the polymer in said emulsion system are determined by means of fluorescence microscopy.

Preferably, the above-described method further comprises after the step C2a):
C2b) adjusting the measured fluorescent image with a predetermined partition factor.

Preferably, in the method of the present invention, further comprising after the step B):
B') centrifuging the system.

After triggering phase separation, many small condensates appear all over the solution and need up to hours to sediment and coarsen. Small condensates are difficult to detect. Therefore, the system is centrifuged to ensure that all condensates fuse to a big condensate in shorter time to facilitate detection.

Preferably, in the step E) of the method of the present invention, at least one condition of said system is selected from a concentration of a component in the system wherein the component is selected from a salt, a crowding agent, or a buffer; the pH value; the pressure; or the temperature of the system, or a combination of the aforementioned conditions.

In some aspects, the present invention refers to an assay method for identifying bioactive compound(s), comprising:

A) determining a total volume ($V_{tot}$) of the system and a total concentration ($c_{tot}$) of a polymer in the system;
A')adding a predetermined amount of test compounds into said system;
B) triggering phase separation of the polymer into a condensed phase and a dilute phase;
C) determining a volume fraction ($V_{con}/ V_{tot}$) of the condensed phase of the polymer in said system;
D) determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

F) identifying the bioactive compounds from the test compounds; and a device comprises a plurality of systems.

Preferably, the polymer is a protein, or a polynucleic acid, preferably RNA or DNA, or a mixture of a protein and RNA, or a mixture of a protein and DNA.

Preferably, the assay method of the invention further comprises after step D):

D') comparing the measured condensed volume ($V_{con}$), the measured condensed concentration ($c_{con}$), the measured dilute concentration ($C_{dil}$), and/or the measured volume fraction ($V_{con}/V_{tot}$) of said polymer in the system with a condensed volume ($V_{ref}$), a condensed concentration ($c_{ref}$), and/or a volume fraction ($V_{ref}/V_{tot}$) of the polymer in the presence of a reference molecule in a control system.

Preferably, in the assay method of the invention, the condensed volume ($V_{ref}$), the condensed concentration ($c_{con\text{-}ref}$), the dilute concentration ($c_{dil\text{-}ref}$) and/or the volume fraction ($V_{ref}/V_{tot}$) of the polymer in the presence of a reference molecule is(are) predetermined or measured at the same time.

Preferably, in the assay method of the invention, the identified bioactive compound(s) increase(s)/decrease(s) the measured condensed volume ($V_{con}$), the measured condensed concentration ($c_{con}$), the measured dilute concentration ($c_{dil}$), and/or the measured volume fraction ($V_{con}/V_{tot}$) of the polymer obtained by the step C) and/or D) than the predetermined condensed volume ($V_{ref}$), the measured condensed concentration ($c_{con\text{-}ref}$), the measured dilute concentration ($c_{dil\text{-}ref}$) and/or the measured volume fraction ($V_{ref}/V_{tot}$) of the polymer of said polymer in the presence of a reference molecule in the control system.

Preferably, in the assay method of the invention, the device is a multi-well plate and each system is contained in each of the wells in a multi-well plate.

In some aspects, the present invention refers to use of the methods according to the invention for determining an optimal condition for crystallization of said polymer.

In some aspects, the present invention refers to a device for determining a binodal curve of a polymer in a system comprising:

i) means for measuring a total volume ($V_{tot}$) of the system;
ii) means for measuring a volume fraction ($V_{con}/V_{tot}$) of a condensed phase polymer in said system;
iii) means for calculating a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}}.$$

Preferably, the polymer is a protein, or a polynucleic acid, preferably RNA or DNA, or a mixture of a protein and RNA or a mixture of a protein and DNA.

An exemplary setup of the inventive device is shown in **Figure 10.** The device utilizes the light reflection at the interface between two media of different refractive index to calculate the height of the condensed phase inside a sample container of defined volume, typically a well in a well-plate.

The device comprises a light source, a dichroic mirror, a pinhole or a slit, a stage, an objective, a z-drive (a motorized focus drive), and a sample container having a defined volume. The light source is preferably an infrared light source (LED or laser) and it is directed to a high N.A objective using the dichroic mirror located behind the pinhole or the slit. At least the stage or the objective needs to be mounted on the z-drive in order to focus the light at different samples depths. In one embodiment, the stage and the objective are mounted on the z-drive. When the light is focused at the container-condensate or at the condensate-solution interfaces, light refraction will be maximal. Light intensity during the scan will be detected and the distance between the peaks corresponds to the height of the condensate phase. With the condensate phase height inside a container of known area, the volume fraction of the condensed phase can then be calculated.

**Detailed Description of the Invention**

Definitions

- **Phase:** an entity of a material system which is uniform in chemical composition and physical rate.
- **Phase separation:** The process by which a single phase separates into two or more new phases.
- **Phase transition:** a change in the nature of a phase or in the number of phases as a result of some variation in

externally imposed conditions, such as temperature, pressure, ionic strength or activity of a component

- **Binodal curve:** also known as coexistence curve, the locus of the compositions of two co-existing phases on a phase diagram. For example, in a temperature-composition plot the binodal curve has a maximum at the upper critical solution temperature, and/or a minimum at the lower critical solution temperature.

- **Phase diagram:** For binary mixtures, the binodal curve is often represented by means of phase diagrams in which the system composition is plotted along the x-axis in the form of the molar fraction of one of the components as the state variable in which the coexisting phases differ. Along the y-axis, an intensive state variable, such as pressure or temperature, is plotted, which must have the same value in the coexisting phases that are in thermodynamic equilibrium. In the case of ternary mixtures, the binodal curve can be projected into a triangular diagram that represents the composition of the ternary mixture.

- **Tie-line:** The line in a phase diagram joining the compositions of independent co-existing phases.

- **Condensate:** the dense phase of a phase separated protein solution. "Dense phase" and "condensed phase" are synonymously used herein.

- **Liquid-liquid phase separation (LLPS):** demixing of a protein solution into a dilute and a dense protein phase (condensates).

- **$c_{con}$ ( $c_{in}$):** protein concentration in the dense phase after phase separation. In a phase diagram it represents the right branch of the binodal.

- **$c_{dil}$ ($c_{out}$)**: protein concentration in the dilute phase after phase separation. It is also the minimum concentration required to enter the demixed state of the binodal. In a phase diagram it represents the left branch of the binodal.

- **Volume fraction:** ratio of the volumes of the total condensed phase over the total volume of the reaction (in case of emulsions the volume of one emulsion droplet).

- **Water-in-oil emulsion:** the resulting emulsion when an aqueous phase is mixed in the presence of an oil and a surfactant.

- **Labelled protein:** fluorescent tag that is added to a protein. This can be directly at the expression of the protein (GFP, RFP, HALO tags plus fluorophore) or via chemical means and a fluorophore (a small fluorescent molecule, for example, Alexa fluorophores with NHS ester).

- **Segmentation:** part of an image analysis that allows to define the regions of interest in an image (e.g. the condensates).

- **Polymer:** The term "polymer" as used herein refers to any macromolecule consisting of repeating units, which is able to undergo liquid-liquid phase separation under specific conditions in solution, i.e. de-mixing into two distinct liquid phases (a condensed and a dilute phase) with different polymer concentrations. Suitable polymers are for example, but not limited to, proteins or acids, including RNA or DNA.

- **Salts:** Salts as used herein refers to any physiologically acceptable salt, including, but not limited to, sodium chloride, sodium fluoride, sodium iodide, potassium chloride, potassium iodide, potassium fluoride, magnesium chloride, calcium chloride, calcium fluoride, cesium chloride, acetate salts, and formate salts.

- **Crowding agents:** Crowding agents as used herein are compounds that enhance phase separation of the polymer, by for instance increasing the effective polymer concentration or by changing the water activity in the system. Suitable crowding agents are for example, but not limited to, macromolecular compounds, such as PEG, dextran, Ficoll™ (an uncharged, highly branched polymer formed by the co-polymerisation of sucrose and epichlorohydrin), bovine serum albumin, or polystyrene sulfonate, or sugar based cosolvents, including trehalose, sucrose, sorbitol, and glycerol.

- **Buffers:** Any buffer used in biochemistry is suitable for the present invention, such as tris(hydroxymethyl)aminomethane (TRIS), 4-(2-hydroxyethyl)-1-piperazineethanesulfonic acid (HEPES), 3-(N-morpholino)propanesulfonic acid

(MOPS), 3-[(3-Cholamidopropyl)dimethylammonio]-1-propanesulfonate (CHAPS) or 2-(*N*-morpholino)ethanesulfonic acid (MES); physiological buffers whose pKs ranges from 7.37 - 7.42 or inorganic buffers like bicarbonate buffers or phosphate buffers.

The present invention provides a method for determining a concentration ($c_{con}$) of a polymer in a condensed phase and a concentration ($c_{dil}$) of the polymer in a dilute phase in a system comprising:

A) determining a total volume ($V_{tot}$) of the system and a total concentration ($c_{tot}$) of the polymer;
B) triggering phase separation of the polymer into a condensed phase and a dilute phase in said system;
C) determining a volume fraction ($V_{con}$ / $V_{tot}$) of the condensed phase of the polymer in said system;
D) determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) changing at least one condition of said system and repeating the steps A) to D).

Preferably, the polymer is a protein, or a polynucleic acid, preferably RNA or DNA, or a mixture of a protein and RNA or a mixture of a protein and DNA.

RNA

In an embodiment of the present invention the method for determining a concentration ($c_{con}$) of a first polymer and a second polymer in a condensed phase and a concentration ($c_{dil}$) of the first polymer and the second polymer in a dilute phase in a system comprises:

A) determining a total volume ($V_{tot}$) of the system and a total concentration ($c_{tot}$) of the first polymer and the second polymer;
B) triggering phase separation of the first polymer into a condensed phase and a dilute phase in said system;
C) determining a volume fraction ($V_{con}$ / $V_{tot}$) of the condensed phase of the first polymer and the second polymer in said system;
D) determining a concentration ($c_{con}$) of the condensed phase of the first polymer and the second polymer and a concentration ($c_{dil}$) of the dilute phase of the first polymer and the second polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) changing at least one condition of said system and repeating the steps A) to D),

wherein the first polymer is a protein and the second polymer is a ribonucleic acid.

Reworded, in an embodiment of the present invention the method for generating a binodal curve of a first polymer and a second polymer in a system comprises:

A) determining a total volume ($V_{tot}$) of the system and a total concentration ($c_{tot}$) of the first polymer and the second polymer;
B) triggering phase separation of the first polymer into a condensed phase and a dilute phase in said system;
C) determining a volume fraction ($V_{con}$ / $V_{tot}$) of the condensed phase of the first polymer and the second polymer in said system;
D) determining a concentration ($c_{con}$) of the condensed phase of the first polymer and the second polymer and a concentration ($c_{dil}$) of the dilute phase of the first polymer and the second polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) changing at least one condition of said system and repeating the steps A) to D),

wherein the first polymer is a protein and the second polymer is a ribonucleic acid.

Preferably, in the method of the invention, the system is *in vitro* system and selected from a solution, an emulsion, or cells.

The term "emulsion" preferably refers to "water-in-oil emulsion" as defined above.

Preferably, in the step B) of the method of present invention, the phase separation of the polymer in said system is triggered by changing the concentration of a salt in the system, the pH value or the temperature of the system.

Preferably, in the method of the invention, when the polymer is not labelled with fluorophore or a fluorescent protein, in step A)

the total volume ($V_{tot}$) of said system and the total concentration ($c_{tot}$) of the polymer are determined by means of bright-field, dark-field, phase-contrast, holographic, polarization, or differential interference correlation (DIC) microscopy, or light-scattering based approaches.

Preferably, in the method of the invention, when the polymer is labelled with a fluorophore or a fluorescent protein, in the step A)

the total volume ($V_{tot}$) of said system and the total concentration ($c_{tot}$) of the polymer are determined by means of a fluorescence microscopy.

As used herein the term "fluorophore" refers to a molecule, label or moiety that is able to absorb energy from light, to transfer this energy internally and emit said energy as light having a specific range of wavelength.

Any of the commercially available fluorophores can be used, for examples, Alexa Fluor® 350, Alexa Fluor® 405, Alexa Fluor®430, Alexa Fluor® 488, Alexa Fluor® 500, Alexa Fluor® 514, Alexa Fluor® 532, Alexa Fluor® 546, Alexa Fluor® 555, Alexa Fluor® 568, Alexa Fluor® 594, Alexa Fluor® 610, Alexa Fluor® 633, Alexa Fluor® 635, Alexa Fluor® 647, Alexa Fluor® 660, Alexa Fluor® 680, Alexa Fluor® 700, Alexa Fluor® 750, fluoresceine, rhodamine B, rhodamine Red, Texas Red®, Cy3, Cy 3.5, Cy5, Cy5.5, Cy7, TRITC, FITC, ATTO-family, abberior-dyes, coumarine-derived dyes.

Preferably, the fluorophore contains any one of the following moieties (**F-1**) - (**F-14**),

| | |
|---|---|
| 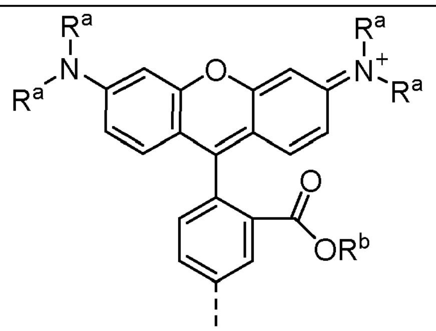 (**F-1**) | 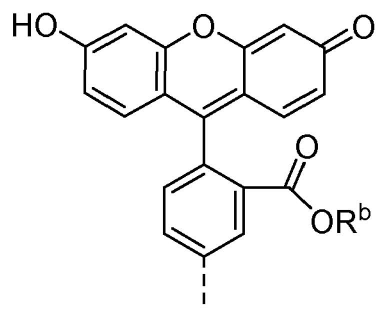 (**F-2**) |
| 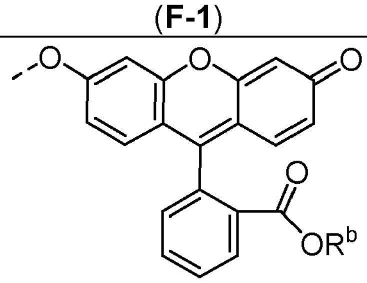 (**F-3**) | 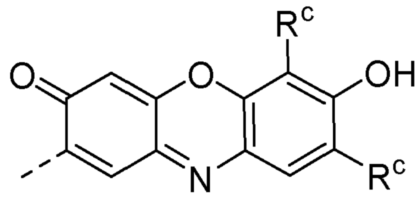 (**F-4)** |
| 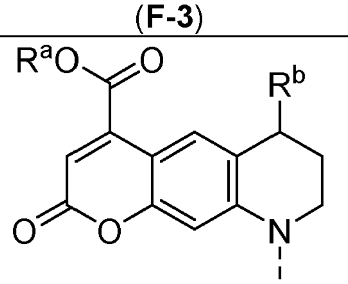 (**F-5**) | 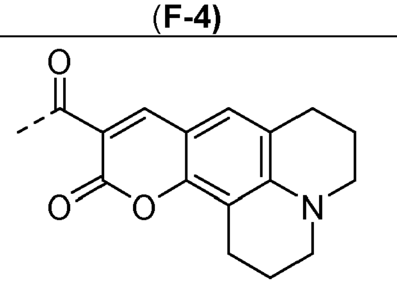 (**F-6**) |
| 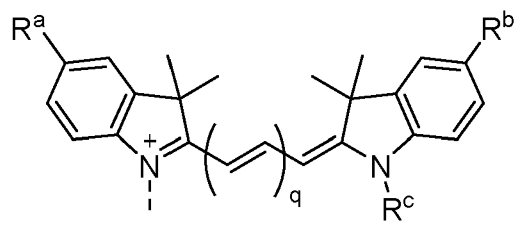 (**F-7**) | 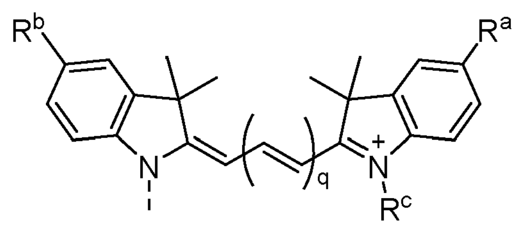 (**F-8**) |

| | |
|---|---|
| 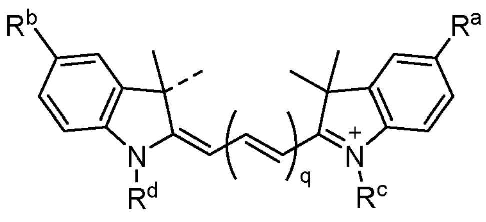 (**F-9**) | 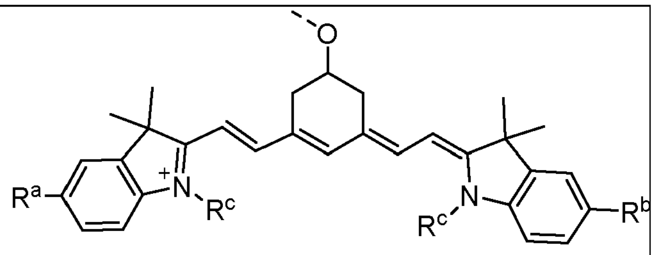 (**F-10**) |
| 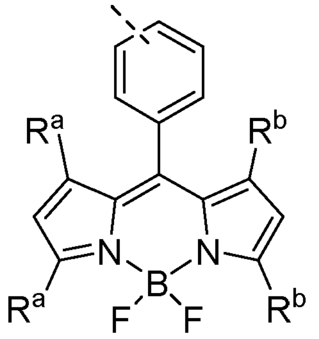 (**F-11**) | 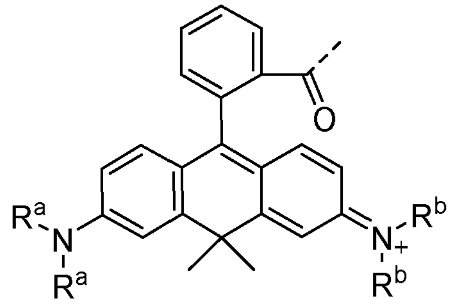 (**F-12**) |
| 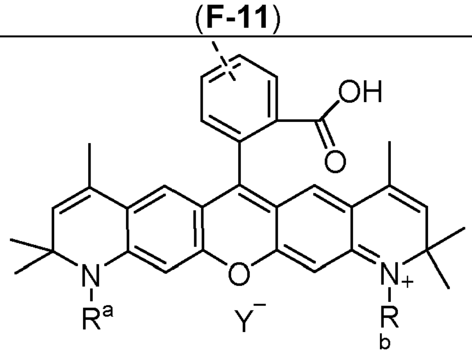 (**F-13**) | 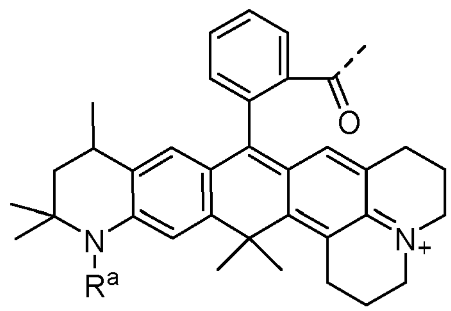 (**F-14**) |

wherein

**$R^a$** and **$R^b$** represent independently of each other -H, $-SO_3H$, $-SO_3^-$, or $-SO_3Na$;
**$R^c$** and **$R^d$** represent independently of each other $-CH_3$, $-C_2H_5$, $-C_3H_7$, $-C_4H_9$, $-C_3H_6-SO_3^-$, or $-C_4H_8-SO_3^-$;
q is an integer selected from 1, 2 and 3.

More preferably, the fluorophore contains any one of the following moieties **(F-7), (F-8),** and/or **(F-9):**

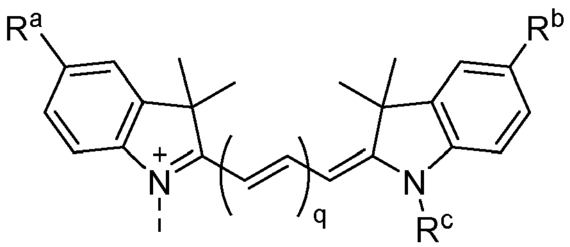

(**F-7**)

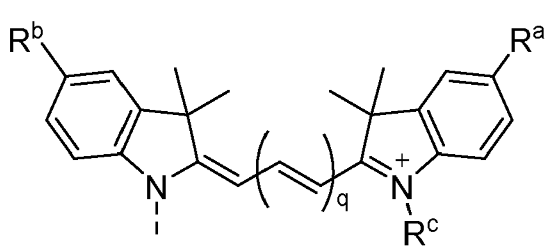

(**F-8**)

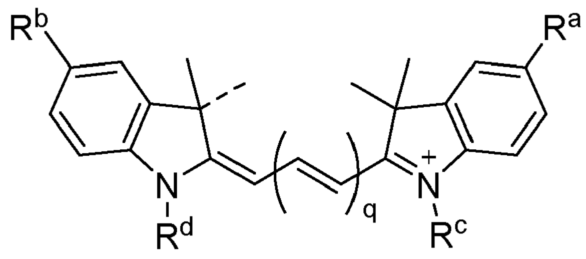

(**F-9**)

wherein

**R**[a] and **R**[b] represent independently of each other -H, $-SO_3H$, $-SO_3^-$, or $-SO_3Na$;
**R**[c] and **R**[d] represent independently of each other $-CH_3$, $-C_2H_5$, $-C_3H_7$, $-C_4H_9$, $-C_3H_6-SO_3^-$, or $-C_4H_8-SO_3^-$;
q is an integer selected from 1, 2 and 3.

More preferably, the fluorophore contains any one of the moieties **(F-7), (F-8),** and/or **(F-9),** wherein **R**[a] and **R**[b] represent independently of each other -H; **R**[c] and **R**[d] represent independently of each other $-CH_3$, $-C_2H_5$, or $-C_3H_7$.

Still more preferably, the fluorophore contains the following moiety:

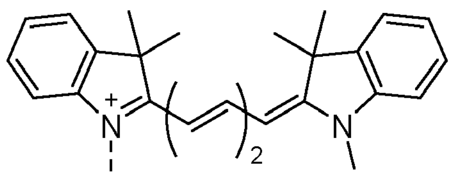

The term "a fluorescent protein" refers to a protein that absorbs light energy of a specific wavelength and re-emits light at a longer wavelength. Presented in **Table 1** is a compilation of properties displayed by several of the most popular and useful fluorescent protein variants. Along with the common name and/or acronym for each fluorescent protein, the peak absorption and emission wavelengths (given in nanometers), molar extinction coefficient, and quantum yield are listed.

Preferred herein are green fluorescent proteins, such as GFP (green fluorescent protein). GFP is a versatile biological marker for monitoring physiological processes, visualizing protein localization, and detecting transgenic expression *in vivo.* GFP can be excited by the 488 nm laser line and is optimally detected at 510 nm

**Table 1 - Fluorescent Protein Properties**

| Protein | Excitation Maximum (nm) | Emission Maximum (nm) | Molar Extinction Coefficient | Quantum Yield |
|---|---|---|---|---|
| Green Fluorescent Proteins | | | | |
| GFP (wt) | 395/475 | 509 | 21,000 | 0.77 |
| EGFP | 484 | 507 | 56,000 | 0.60 |
| Emerald | 487 | 509 | 57,500 | 0.68 |
| Superfolder GFP | 485 | 510 | 83,300 | 0.65 |
| Azami Green | 492 | 505 | 55,000 | 0.74 |
| mWasabi | 493 | 509 | 70,000 | 0.80 |
| TagGFP | 482 | 505 | 58,200 | 0.59 |
| TurboGFP | 482 | 502 | 70,000 | 0.53 |
| AcGFP | 480 | 505 | 50,000 | 0.55 |
| ZsGreen | 493 | 505 | 43,000 | 0.91 |
| T-Sapphire | 399 | 511 | 44,000 | 0.60 |

(continued)

| | | | | |
|---|---|---|---|---|
| Blue Fluorescent Proteins | | | | |
| EBFP | 383 | 445 | 29,000 | 0.31 |
| EBFP2 | 383 | 448 | 32,000 | 0.56 |
| Azurite | 384 | 450 | 26,200 | 0.55 |
| mTagBFP | 399 | 456 | 52,000 | 0.63 |
| Cyan Fluorescent Proteins | | | | |
| ECFP | 439 | 476 | 32,500 | 0.40 |
| mECFP | 433 | 475 | 32,500 | 0.40 |
| Cerulean | 433 | 475 | 43,000 | 0.62 |
| mTurquoise | 434 | 474 | 30,000 | 0.84 |
| CyPet | 435 | 477 | 35,000 | 0.51 |
| AmCyanl | 458 | 489 | 44,000 | 0.24 |
| Midori-Ishi Cyan | 472 | 495 | 27,300 | 0.90 |
| TagCFP | 458 | 480 | 37,000 | 0.57 |
| mTFP1 (Teal) | 462 | 492 | 64,000 | 0.85 |
| Yellow Fluorescent Proteins | | | | |
| EYFP | 514 | 527 | 83,400 | 0.61 |
| Topaz | 514 | 527 | 94,500 | 0.60 |
| Venus | 515 | 528 | 92,200 | 0.57 |
| mCitrine | 516 | 529 | 77,000 | 0.76 |
| YPet | 517 | 530 | 104,000 | 0.77 |
| TagYFP | 508 | 524 | 64,000 | 0.60 |
| PhiYFP | 525 | 537 | 124,000 | 0.39 |
| ZsYellow1 | 529 | 539 | 20,200 | 0.42 |
| mBanana | 540 | 553 | 6,000 | 0.7 |
| Orange Fluorescent Proteins | | | | |
| Kusabira Orange | 548 | 559 | 51,600 | 0.60 |
| Kusabira Orange2 | 551 | 565 | 63,800 | 0.62 |
| mOrange | 548 | 562 | 71,000 | 0.69 |
| mOrange2 | 549 | 565 | 58,000 | 0.60 |
| dTomato | 554 | 581 | 69,000 | 0.69 |
| dTomato-Tandem | 554 | 581 | 138,000 | 0.69 |
| TagRFP | 555 | 584 | 100,000 | 0.48 |
| TagRFP-T | 555 | 584 | 81,000 | 0.41 |
| DsRed | 558 | 583 | 75,000 | 0.79 |
| DsRed2 | 563 | 582 | 43,800 | 0.55 |
| DsRed-Express (T1) | 555 | 584 | 38,000 | 0.51 |
| DsRed-Monomer | 556 | 586 | 35,000 | 0.10 |

(continued)

| | | | | |
|---|---|---|---|---|
| Orange Fluorescent Proteins | | | | |
| mTangerine | 568 | 585 | 38,000 | 0.30 |
| Red Fluorescent Proteins | | | | |
| mRuby | 558 | 605 | 112,000 | 0.35 |
| mApple | 568 | 592 | 75,000 | 0.49 |
| mStrawberry | 574 | 596 | 90,000 | 0.29 |
| AsRed2 | 576 | 592 | 56,200 | 0.05 |
| mRFP1 | 584 | 607 | 50,000 | 0.25 |
| JRed | 584 | 610 | 44,000 | 0.20 |
| mCherry | 587 | 610 | 72,000 | 0.22 |
| HcRed1 | 588 | 618 | 20,000 | 0.015 |
| mRaspberry | 598 | 625 | 86,000 | 0.15 |
| dKeima-Tandem | 440 | 620 | 28,800 | 0.24 |
| HcRed-Tandem | 590 | 637 | 160,000 | 0.04 |
| mPlum | 590 | 649 | 41,000 | 0.10 |
| AQ143 | 595 | 655 | 90,000 | 0.04 |

In some aspects, in step C) of the method of the present invention, step C) comprises:

C1a) encapsulating a portion of the system in an emulsion system immediately;
C2a) determining a volume ($V_{em}$) of one emulsion droplet of the emulsion system, and a condensed volume ($V_{con}$') of the condensed phase of the polymer in said emulsion system;
C3a) determining a volume fraction ($V_{con'}/V_{em}$) of the condensed volume ($V_{con}$') of the condensed phase of the polymer in said system and the volume ($V_{em}$) of one emulsion droplet of said emulsion system, wherein the measured volume fraction ($V_{con'}/V_{em}$) is identical to the volume fraction ($V_{con}/V_{tot}$) of the condensed volume ($V_{con}$) of the condensed phase of the polymer in the system and the total volume ($V_{tot}$) of the system.

Preferably, the emulsion system is a water-in-oil emulsion system. Thus step C) of the methods described herein preferably comprises:

C1a) encapsulating a portion of the system in a water-in-oil emulsion system immediately;
C2a) determining a volume ($V_{em}$) of one emulsion droplet of the water-in-oil emulsion system, and a condensed volume ($V_{con}$') of the condensed phase of the polymer in said water-in-oil emulsion system;
C3a) determining a volume fraction ($V_{con'}/V_{em}$) of the condensed volume ($V_{con}$') of the condensed phase of the polymer in said water-in-oil emulsion system and the volume ($V_{em}$) of one emulsion droplet of said water-in-oil emulsion system, wherein the measured volume fraction ($V_{con'}/V_{em}$) is identical to the volume fraction ($V_{con}/V_{tot}$) of the condensed volume ($V_{con}$) of the condensed phase of the polymer in the system and the total volume ($V_{tot}$) of the system.

Preferably, in the step C2a), a volume ($V_{em}$) of the emulsion system, and a condensed volume ($V_{con}$') of the condensed phase of the polymer in said emulsion system are determined by means of a fluorescence microscopy.
Preferably, the above-described method further comprises after the step C2a):
C2b) adjusting the measured fluorescent image with a predetermined partition factor.
The term "partition factor (PF)" is often used in condensate research to characterise the up-concentration of protein in the condensed phase. It can be defined by the ratio of the condensed- over dilute-phase concentration ( $PF = c_{con} / c_{dil}$ ). In the condensate field it is common to determine the partition factor by comparing fluorescence intensity inside the condensate against the intensity in the dilute phase. In contrast, the inventive method allows to directly determining the partition factor by using the presented phase diagrams (Fig. 2).
Preferably, in the method of the present invention, further comprising after the step B):

B') centrifuging the system.

The polymer may be labeled with a fluorophore or a fluorescent protein or not.

Preferably, the height of the condensed phase film is detected/determined by means of light refraction between the surface of the condensed and solute phase. Surface detecting devices are already available in the market such as the Nikon and Olympus perfect focus and ZDC.

Alternatively, capillaries can be scanned by a laser beam to detect the surface between condensed and solute phase.

Preferably, in the step E) of the method of the present invention, the at least one condition of said system is selected from a concentration of a component in the system wherein the component is selected from a salt, a crowding agent, or a buffer; the pH value; the pressure; a crowding agent; or the temperature of the system, or a combination of the aforementioned conditions.

Salt and temperature dependent phase diagrams of two well studied proteins, fused in sarcoma (FUS) and *C. elegans'* P granule protein PGL-3, are demonstrated to validate the inventive method. The results are in good agreement with standard bulk methods. It is proven that phase diagrams produced by the inventive method provide novel insight into the phase behaviour of these proteins (Figure1). The phase diagrams are tested by temperature shift experiments that cross the derived binodal and enter the two-phase region. Finally, it was demonstrated that inventive method is accurate enough to differentiate small changes in the phase diagram caused by the addition of small molecules.

The ability to measure the (temperature dependent) binodal curve for a given polymer furthermore enables comparison to various physical theories and simulation methods. These include models like Flory-Huggins and its extensions to Voorn-Overbeek theory, field theoretic simulations, or random phase approximation. In comparing the experimental data with the predictions of these models, an appropriate physical model to describe the thermodynamics of a given phase separating polymer can be identified. Furthermore, this allows access to determining model parameters like the interaction strength between individual polymers.

**Concept of the inventive method (inPhase).** If a protein phase separates in a closed system, the total volume, $V_{tot}$ , is conserved and it can be expressed as the sum of the volumes of the dilute phase and the condensed phase ($V_{tot} = V_{dil} + V_{con}$, Fig. 1b). In this closed system, the total protein mass is also conserved ( $m_{tot} = m_{dil} + m_{con}$ ); given that the mass equals the volume times the concentration ($m = V \cdot c$), we can express the previous equation as $V_{tot}\, c_{tot} = V_{dit}\, c_{dil} + V_{con}\, c_{con}$. Rearranging the volume and mass conservation equations leads to:

$$\frac{V_{con}}{V_{tot}} = \frac{1}{c_{con} - c_{dil}} \cdot c_{tot} - \frac{c_{dil}}{c_{con} - c_{dil}} \qquad \text{(Eq. 1)}$$

Thus, it is shown that the ratio $V_{con}/V_{tot}$, referred to as the volume fraction, changes linearly with the total protein concentration, $c_{tot}$, of the system **(Figure 1g).** A linear regression to a concentration series allows us to determine both $c_{dil}$, via the x-intercept, and $c_{con}$ by using the slope of the line, given by $(c_{con} - c_{dil})^{-1}$. It therefore follows that we can determine $c_{con}$ and $c_{dil}$ at defined external conditions (temperature, salt, pH) by preparing samples at different total protein concentrations, $c_{tot}$, and by measuring the volume of the condensed phase, $V_{con}$, in a closed system of known volume, $V_{tot}$. In principle, this allows us to measure binodal curves for ordinary binary systems.

To set a closed environment, a water-in-oil emulsion system is used, where the aqueous solution is encapsulated in stable emulsion droplets (Fig. 1c). Figure 1d shows a typical experimental scheme, where a dilution series of $c_{tot}$ is mounted to a temperature-controlled stage. The volume of individual emulsion droplets can be detected from bright-field images by assuming that the droplets form nearly spherical containers (Fig. 1d and 1e). Finally, the protein phase volume, $V_{con}$, is measured by image segmentation and analysis of microscopy images (Fig. 1e). The presence of tagged-protein is not strictly necessary but facilitates the segmentation.

This allows now to test the linear relationship between volume fraction and total concentration for *in vitro* protein solutions. The image analysis pipeline was calibrated with fluorescent polystyrene beads of known size since the inventive technique described herein relies on an accurate determination of the volume of the condensed phase (Figure 1h). Figure 1f shows the result of applying the method to FUS::GFP at 150 mM KCl at 15 and 25 °C. The inventors observed that the linear regression is in good agreement with our experimental data.

**Acquiring binodal curves with the inventive method (inPhase).** The widely used fused in sarcoma (FUS) protein and PGL-3, a protein component of P granules found in the germline of *C. elegans* were chosen as examples to measure their temperature and salt dependant binodal curves. For PGL-3 it has been shown *in vivo* that the protein concentration necessary for phase separation increases with increasing temperatures (Fritsch, A. W. et al. Local thermodynamics govern formation and dissolution of Caenorhabditis elegans P granule condensates. PNAS, 2021, 118(37), e2102772118). A dilution series of PGL-3 at different temperatures recapitulates this behaviour *in vitro* (Fig. 2a). This representation of imaging data gives a visual impression at which protein concentration phase separation is triggered for different temperatures (indicated by a dashed line).

The binodal curves of the phase diagrams can be derived after segmentation and analysis of the microscopy data (Fig. 2 b-e). The inventors found that the dilute branch concentration $c_{dil}$ increases for higher salt concentrations as well as for higher temperatures (Fig. 2b, c). Comparing FUS and PGL-3 at the same salt concentration we observe that FUS requires less protein for phase separation, it reaches higher concentrations in the condensed phase and its $c_{dil}$ is more sensitive to temperature changes. The different sensitivities of both proteins to temperature could indicate a different mechanism for phase separation.

For the presented temperature phase diagrams, a critical temperature was not observed (Fig. 1a and Fig. 2b, c). This is because increasing the temperature results in aggregation rather than phase dissolution (Extended Data Fig. 4). This is indicated by the bending that appears on the condensed branch of the binodal towards higher protein concentrations for higher temperatures. Interestingly, the condensed branch bending is more prominent and starts at lower temperatures for higher salt concentrations. To discard artefacts from starting with cold samples and increasing the temperatures, we also measured the binodal curves starting from warm samples and cooling down. The temperature changes give similar results in either direction (Extended Data Fig. 5).

Similar to the case for temperature, an increase in salt concentration results in an increase in $c_{dil}$ (Fig. 2d, e). Here, a stronger effect of salt concentration for PGL-3 on $c_{dil}$ compared to FUS could be seen. These findings are summarized in the 3D plot of the dilute branch surfaces against temperature and salt concentration for both proteins (Fig. 2f). Furthermore, a higher sensitivity of $c_{dil}$ to salt concentration when compared to temperature changes, especially for PGL-3 was observed. This difference can be seen by the rather box-shaped phase diagrams in Fig. 2b as compared to Fig. 2d. The different sensitivities of the phase diagrams between temperature and salt can be further used to study the molecular interactions that determine phase separation. For example, a higher sensitivity to salt as compared to temperature suggests that ionic interactions are important for phase separation in the case of PGL-3.

**Testing binodals with dynamic temperature shifts.** The inventors harbor the reversibility of phase separation to assess the validity of our $c_{dil}$ estimates. For these experiments FUS was chosen because of its higher sensitivity to temperature changes. From the temperature phase diagrams of FUS at different salt concentrations, we can determine a protein concentration that allows us to move in and out of the demixed region with temperature (Fig. 3a). Samples at 100, 150, 200 or 300 mM KCl that contained 1.5, 3, 5 or 10 μM of FUS, respectively were prepared. From the phase diagrams it was expected that all the samples will phase separate at similar temperatures, termed condensation temperature $T_{cond}$. Starting from 30 °C the temperature was reduced to 5 °C as depicted in Fig 3b. As expected, all protein mixes started to phase separate around the predicted temperature (indicated by the black region in graph in Fig 3b). The slightly lower $T_{cond}$ for 300 mM KCl compared to 100 mM KCl is also reflected in a later entry into the phase separated region in the dynamic experiment. While the higher salt concentrations dissolve all condensates after heating to the initial temperature, for the lower salt concentrations small remnants are still visible after the temperature quench (orange dashed line Fig 3b). It was observed that the measured dilute-branch binodals are in good agreement with the presented temperature quenches. This showcases the benefit of such a detailed understanding of a protein system for further experimental work on dynamic quenches into the demixed region.

**Extension and further verification of inventive method (inPhase).** Herein phase diagrams for two proteins are presented that were derived using the inventive method on a water-in-oil emulsion system. The method is however not limited to the use of emulsions that could be also generated in a microfluidic system, but can be applied wherever the volume-fraction of a condensed protein can be determined. A well-plate format was used as a scalable system that can be extended to screening approaches. In this case the total volume of the sample per well is known and the volume of the condensate phase can be determined via fluorescence. In Figure 4a, such an experiment for PGL-3 is presented, as can be seen the total condensate volume increases with increasing total protein concentration.

Next, the method in its different modes (fluorescence and brightfield segmentation in emulsions, and fluorescence segmentation in well-plates) was validated by directly measuring the protein concentration in the dilute and condensed phase via light absorbance and the BCA (bicinchoninic acid) assay (Fig. 4b, c). The results show that the inventive method outputs accurate values for both the dilute and the condensed phase concentrations. While the well-plate assay in the current iteration is less precise, the obtained average is close to the values of the other measurements. The discrepancy of the absorbance measurement for the dilute phase to all other measurements can be explained by the low protein concentrations, which lies at the detection limit of the nanoDrop device.

**Partition factor and comparison to fluorescence-based quantification.** The partition factor (PF) is often used in condensate research to characterise the up-concentration of protein in the condensed phase. It can be defined by the ratio of the condensed- over dilute-phase concentration *($PF = c_{con} / c_{dil}$)*. In the condensate field it is common to determine the partition factor by comparing fluorescence intensity inside the condensate against the intensity in the dilute phase. In contrast, our method allows us to directly determine the partition factor by using the presented phase diagrams (Fig. 2).

For FUS, it was observed that partition factors in the order of $10^2$-$10^3$ strongly depend on the temperature and salt concentration (Fig. 5a). The partition factor of FUS exhibits a minimum that can be observed at lower temperatures with increasing salt concentrations. This minimum corresponds to the onset of bending of the condensed branch as

presented in the phase diagrams in Fig. 2c, it is therefore a consequence of increased FUS concentration in the condensed phase. For PGL-3 only a small influence of temperature was observed while salt concentration can change the partition factor by an order of magnitude between $10^1$-$10^2$ (Fig. 5b). This trend is only broken for higher temperatures and salt concentrations where a sharp increase in the partition factor takes place. It is hypothesized that these changes in partitioning factor follow from a change in protein-protein interactions and protein secondary structure.

Next, calculated partition values are compared to the ones obtained with commonly used quantitative fluorescence intensity approach. Given that fluorescence was also used to segment the condensate volume, the same data can be used to directly compare both methods ($PF_I$, via intensity, $PF_c$ via inventive method). The inventors found a strong dependence of the quantitative intensity-derived partition factor $PF_I$ on condensate size for both FUS and PGL-3 (Fig. 5d). Above a condensate volume of approximately $10^2$ $\mu m^3$ the partition factor $PF_I$ levels off to a plateau-like value that is however still smaller than the value we calculate via the method disclosed herein (orange dashed line). A value for $PF_I$ for a given external condition can be calculated by only using condensates above a threshold volume that is in the plateau region. In general, the $PF_I$ underestimates the protein partitioning (Fig. 5e). The difference between $PF_I$ and $PF_c$ is more prominent for FUS than for PGL-3, and at least 2-fold off over the entire dataset. This effect is likely caused by an erroneous estimation of the fluorescence intensity inside the condensate phase. These could be caused by limitations of the optical system as well as by a different chemical environment inside the phase that changes the fluorescence properties. To this end, the inventors tested the influence of the fraction of labelled protein on the partition factor PF, and $PF_c$ for PGL-3. While an increase in the partition factor is seen for increased fraction of labelled protein for both $PF_I$ and $PF_c$, the ratio $PF_I$ / $PF_C$ is constant over all label fractions tested (Fig. 5c).

**Discussion**

Phase diagrams produced by the inventive method that present the dilute and condensed phase protein concentrations have been reported for phase separating proteins. These phase diagrams contain rich information on the protein behaviour and interactions that can be used to assess current theoretical approaches (cite later). Yet, to realize the full potential of phase separation research better methods for measuring phase diagrams are still required. The inventive method offers a powerful approach to address this challenge. The inventors showed quantitatively that accurate phase diagrams can be generated at defined temperature and salt concentrations for FUS and PGL-3 proteins. Based on these phase diagrams dynamic temperature experiments can be performed to assess the reversibility of phase separation and quantify the effect of small molecules on phase behaviour.

The inventive method is based on mass- and volume-conversation to measure accurate concentrations of the dilute and condensed phase of phase separating proteins. The underlying principles and techniques make this method accessible to the research community because it relies on commonly available equipment (fluorescent or bright field microscope), modest computational power and low amounts of protein. Our approach does not necessitate the use of labelled protein, which not only reduces the time required for cloning and protein purification but also removes artefacts associated with the use of fluorescent tags. Furthermore, the accurate measurement of binodal curves with the inventive method has the potential to increase reproducibility in the protein phase separation community as protein concentrations in and out of the phase can be reported with their experimental errors.

It was demonstrated that the partition factors calculated using the inventive method overcome common issues encountered when using quantitative fluorescence microscopy. For example, given a 16-bit image of a droplet with a fluorescence count of $65 \times 10^3$ in an image that has a dark current signal of 100 counts and with a fluorescent intensity outside the condensate of 150 counts, the maximum partition factor that can be detected is about $1.3 \times 10^3$. As it has been shown, this range is not enough to measure the partitioning factor of FUS, and probably extends to other proteins of the same family. The dynamic range of the camera is not the only limiting factor in quantitative fluorescence but it is well known that fluorescent properties depend on the environment. Given that the chemical environment is different inside the condensate compared to the dilute phase it is not safe to assume a linear relationship of the fluorescent intensity between the condensate and its surroundings.

Other known methods to study the dilute or condensed branch concentrations include more elaborate bulk measurements, measurements of optical density, microfluidic systems, or fluorescence correlation spectroscopy (FCS). While optical density measurements provide a reliable way to determine the condensed branch concentrations as well as a limited possibility to be used in an *in vivo* setting, they do not provide dilute branch concentrations, need dedicated equipment, and are difficult to be converted to be used for screening.

In conclusion, the inventive method is an efficient new method for measuring binodal curves, and it will contribute to developing the study of phase separating proteins. While other techniques have been developed to measure binodal curves, they often require specialised equipment (microfluidic chips) or great amounts of protein and therefore their applicability might be limited. The concept at the core of inventive method is readily accessible to a wide community and that the experimental workflow, performing a protein titration, can be readily adapted.

## Effect of small molecule drugs and screening methods

The methods of the present invention are compared to the widely used quantitative fluorescence approaches and find that these methods underestimate the protein concentration in the condensates for FUS and PGL-3. Thus, the inventive methods can be used in large-scale screening approaches to generate databases of binodal curves, opening the possibility for the thermodynamic assessment of entire protein families and for pharmaceutical studies.

In some aspects, the present invention refers to an assay method for identifying bioactive compound(s), comprising:

A) determining a total volume ($V_{tot}$) of the system and a total concentration ($c_{tot}$) of a polymer in the system;
A') adding a predetermined amount of test compounds into said system;
B) triggering phase separation of the polymer into a condensed phase and a dilute phase
C) determining a volume fraction ($V_{con}$/ $V_{tot}$) of the condensed phase of the polymer in said system;
D)determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

F) identifying the bioactive compounds from the test compounds;

wherein the polymer is a protein, or a polynucleic acid; and a device comprises a plurality of systems.

Preferably, the assay method of the invention further comprises after step D):
D') comparing the measured condensed volume ($V_{con}$), the measured condensed concentration ($c_{con}$), and/or the measured volume fraction ($V_{con}/V_{tot}$) of said polymer in the system with a condensed volume ($V_{ref}$), a condensed concentration ($c_{ref}$), and/or a volume fraction ($V_{ref}/V_{tot}$) of the polymer in the presence of a reference molecule in a control system.

Preferably, in the assay method of the invention, the condensed volume ($V_{ref}$), the condensed concentration ($c_{ref}$), and/or the volume fraction ($V_{ref}/V_{tot}$) of the polymer in the presence of a reference molecule is(are) predetermined or measured at the same time.

Preferably, in the assay method of the invention, the identified bioactive compound(s) increase(s) the measured condensed volume ($V_{con}$), the measured condensed concentration ($c_{con}$), the measured dilute concentration ($c_{dil}$), and/or the measured volume fraction ($V_{con}/V_{tot}$) of the polymer obtained by the step C) and/or D) than the predetermined condensed volume ($V_{ref}$), the measured condensed concentration ($c_{ref}$), and/or the measured volume fraction ($V_{ref}/V_{tot}$) of the polymer of said polymer in the presence of a reference molecule in the control system.

Preferably, in the assay method of the invention, the device is a multi-well plate and each system is contained in each of wells in a multi-well plate.

The bioactive compound is preferably a small molecule (molecular weight < 1000 Da), and more preferably a therapeutically active small molecule, i.e. a small molecule drug.

To demonstrate the possibilities of drug screening, a small molecule (for example, Lipoamide) is used that has been indicated to affect the phase separation of FUS a protein related to ALS. It has so far been difficult to accurately the extent of the effect because of a lack of quantitative measures.

Example 3 shows the effect of lipoamide on the phase behavior of FUS where a reduced amount of conformational changes at higher temperatures was observed. This is indicated by the earlier bent of the condensed phase branch of the binodal for the untreated control (DMSO). This can also be seen in the partition factor $c_{in}$ / $c_{out}$.

## Use of Phase diagrams for determining optimal phase separation conditions

In some aspects, the present invention refers to use of the methods according to the invention for determining an optimal condition for crystallization of said polymer.

The binodal curve is the coexistence curve for thermodynamic stability which a transition occurs from a stable single-phase mixture towards loss of miscibility and subsequent demixing. This results in the formation of a dilute and condensed (dense) polymer liquid phase. Liquid-liquid demixing involves a nucleation step, and as such, requires the passage over an activation barrier.

The spinodal curve describes the limit or loss of this metastability, i.e. barrier free phase separation and instability against all fluctuations. The spinodal curve is of kinetic origin. It defines the transition to a nucleus size of one polymer and has been observed, in particular for proteins. The liquid-liquid critical point is then the intersection of the bimodal and spinodal curves. At the critical point, there is no distinction between the two liquid phases and phase boundaries

cease to exist. Liquid-liquid demixing has been observed experimentally for many proteins. For these cases, the liquid-liquid-coexistence curve is located in the metastable region under the liquid-solid coexistence curve.

Thus, the phase diagram derived from the binodal curves produced by the methods according to the invention is useful for determining an optimal condition for phase separation of said polymer, in particular, proteins.

Phase diagrams ***in vivo***

In general, the method of the present invention can be used for any phase separating system. This is also true for *in vivo* settings of phase separating proteins. The natural or induced fluctuations in the total protein concentration of cells are equivalent to our protein concentration titration series. Thus, the inventive method can be applied to calculate $c_{con}$ and $c_{dil}$. As previously discussed, we determine the volume fraction of the phase and the total concentration of protein (e.g. via GFP labelled protein). This is shown exemplary for *C. elegans* embryos and the protein PGL-1 that is constitutive for P granules.

Since *in vivo* condensates are usually multicomponent systems, the $c_{con}$ can be understood as an average for all proteins in the phase. This opens the possibility to look at the multicomponent nature of condensates, e.g. by concertation titration of one component while keeping the other constant. Furthermore using *in vivo* screens could be used in translational research for drug discovery.

The present invention is directed to an *in vivo* method for the diagnosis of a disease associated with and/or caused by phase separation of a polymer in a cell-containing sample comprising:

A) determining a total concentration ($c_{tot}$) of a phase-separated polymer within a cell contained in said sample;
C) determining a volume fraction ($V_{con}$/ $V_{tot}$) of a condensed phase of the polymer in said cell contained in said sample;
D)determining a concentration ($c_{con}$) of the condensed phase of the polymer and a concentration ($c_{dil}$) of the dilute phase of the polymer in said cell contained in said sample by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) repeating steps A) - D) on a different cell contained in said sample.

Preferably, the polymer is a protein, or a polynucleic acid, preferably RNA or DNA, or a mixture of a protein and RNA or a mixture of a protein and DNA.

Preferably, the disease associated with and/or caused by phase separation of a polymer is cancer, a neurodegenerative disease or an infectious disease. More preferably, the disease is selected from ALS, Parkinson, Alzheimer, Huntington, frontotemporal dementia, cancer, and infectious disease.

A phase-separated polymer refers to a polymer being present in condensed phase and a dilute phase within the system.

In a preferred embodiment the *in vivo* method for the diagnosis of a disease associated with and/or caused by phase separation of a polymer in a cell-containing sample comprising:

A) determining a total concentration ($c_{tot}$) of a phase-separated polymer within a cell contained in said sample;
C) determining a volume fraction ($V_{con}$/ $V_{tot}$) of a condensed phase of the polymer in said cell contained in said sample;
D)determining a concentration ($c_{con}$) of the condensed phase of the polymer and a concentration ($c_{dil}$) of the dilute phase of the polymer in said cell contained in said sample by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) inducing RNA interference to decrease the total concentration ($c_{tot}$) of a phase-separated polymer and repeating steps A) - D),

wherein the polymer is a protein.

In a preferred embodiment the *in vivo* method for the diagnosis of a disease associated with and/or caused by phase separation of a polymer in a cell-containing sample comprising:

A) determining a total concentration ($c_{tot}$) of a phase-separated polymer within a cell contained in said sample;

C) determining a volume fraction ($V_{con}$/ $V_{tot}$) of a condensed phase of the polymer in said cell contained in said sample;
D)determining a concentration ($c_{con}$) of the condensed phase of the polymer and a concentration ($c_{dil}$) of the dilute phase of the polymer in said cell contained in said sample by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) inducing gene expression in said cell to increase the total concentration ($c_{tot}$) of a phase-separated polymer and repeating steps A) - D),

wherein the polymer is a protein.

The present invention is directed to an *ex vivo* method for the diagnosis of a disease associated with and/or caused by phase separation of a polymer in a cell-containing sample comprising:

A1) obtaining a cell-containing sample from a subject,
A2) determining a total concentration ($c_{tot}$) of a phase-separated polymer within a cell contained in said sample;
C) determining a volume fraction ($V_{con}$/ $V_{tot}$) of a condensed phase of the polymer in said cell contained in said sample;
D) determining a concentration ($c_{con}$) of the condensed phase of the polymer and a concentration ($c_{dil}$) of the dilute phase of the polymer in said cell contained in said sample by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) repeating steps A) - D) on a different cell contained in said sample.

Preferably, the polymer is a protein, or a polynucleic acid, preferably RNA or DNA, or a mixture of a protein and RNA or a mixture of a protein and DNA.

Preferably, the disease associated with and/or caused by phase separation of a polymer is cancer, a neurodegenerative disease or an infectious disease. More preferably, the disease is selected from ALS, Parkinson, Alzheimer, Huntington, frontotemporal dementia, cancer, and infectious disease.

In a preferred embodiment the *ex vivo* method for the diagnosis of a disease associated with and/or caused by phase separation of a polymer in a cell-containing sample comprising:

A1) obtaining a cell-containing sample from a subject,
A2) determining a total concentration ($c_{tot}$) of a phase-separated polymer within a cell contained in said sample;
C) determining a volume fraction ($V_{con}$/ $V_{tot}$) of a condensed phase of the polymer in said cell contained in said sample;
D) determining a concentration ($c_{con}$) of the condensed phase of the polymer and a concentration ($c_{dil}$) of the dilute phase of the polymer in said cell contained in said sample by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) inducing RNA interference to decrease the total concentration ($c_{tot}$) of a phase-separated polymer and repeating steps A) - D),

wherein the polymer is a protein.

In a preferred embodiment the *ex vivo* method for the diagnosis of a disease associated with and/or caused by phase separation of a polymer in a cell-containing sample comprising:

A1) obtaining a cell-containing sample from a subject,
A2) determining a total concentration ($c_{tot}$) of a phase-separated polymer within a cell contained in said sample;
C) determining a volume fraction ($V_{con}$/ $V_{tot}$) of a condensed phase of the polymer in said cell contained in said sample;
D)determining a concentration ($c_{con}$) of the condensed phase of the polymer and a concentration ($c_{dil}$) of the dilute phase of the polymer in said cell contained in said sample by using a following linear form equation:

$$\frac{V_{con}}{V_{tot}} = \frac{1}{c_{con} - c_{dil}} \cdot c_{tot} - \frac{c_{dil}}{c_{con} - c_{dil}},$$

E) inducing gene expression in said cell to increase the total concentration ($c_{tot}$) of a phase-separated polymer and repeating steps A) - D),

wherein the polymer is a protein.

Suitable samples for the diagnostic methods described herein are for example, but not limited to, bodily fluids, including blood, urine, cerebrospinal fluid, peritoneal fluid, pleural fluid, ascitic fluid, blood plasma, liver extracts and interstitial fluid.

## Phase volume detection device

The presented approaches to determine the volume fraction of the condensed phase rely on the use of imaging and image segmentation. This has the intrinsic problem of time and cost inefficiency. Herein, alternative approaches to determine the volume fraction using light scattering properties of a surface are presented.

Thus, the present invention is directed to a device for determining a binodal curve of a polymer in a system comprising:

i) means for measuring a total volume ($V_{tot}$) of the system;
ii) means for measuring a volume fraction ($V_{con}/V_{tot}$) of a condensed phase of the polymer in said system;
iii) means for calculating a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con} - c_{dil}} \cdot c_{tot} - \frac{c_{dil}}{c_{con} - c_{dil}},$$

wherein the polymer is a protein, or a polynucleic acid.

Using well-plates that are high binding towards the protein we can generate a film of condensed phase after centrifugation (see **Figure 6).** Thus, a volume fraction ($V_{con}$ / $V_{tot}$) of a condensed phase of the polymer in said system can be determined by detecting the height of the condensed phase film by means of light refraction between the surface of the condensed and solute phase. Thus, surface detecting devices such as the Nikon and Olympus perfect focus and ZDC can be used as means for measuring a volume fraction ($V_{con}$ / $V_{tot}$) of a condensed phase of the polymer in said system.

An exemplary setup of the inventive device is shown in **Figure 10.** The device utilizes the light reflection at the interface between two media of different refractive index to calculate the height of the condensed phase inside a sample container of defined volume, typically a well in a well-plate (see **Figure 11).**

The device comprises a light source, a dichroic mirror, a pinhole or a slit, a stage, an objective, a z-drive (a motorized focus drive), and a sample container having a defined volume. The light source is preferably an infrared light source (LED or laser) and it is directed to a high N.A objective using the dichroic mirror located behind the pinhole or the slit. At least the stage or the objective needs to be mounted on the z-drive in order to focus the light at different samples depths. In one embodiment, the stage and the objective are mounted on the z-drive. When the light is focused at the container-condensate or at the condensate-solution interfaces, light refraction will be maximal. Light intensity during the scan will be detected and the distance between the peaks corresponds to the height of the condensate phase. With the condensate phase height inside a container of known area, the volume fraction of the condensed phase can then be calculated.

Preferably, the sample container has a constant cross-section in height for facilitating the determination of the liquid level and height of the condensed phase film. Preferably, the sample container is of cylindrical shape.

Alternatively, capillaries or capillary tubings or capillary tubes can be used as sample containers. The capillary tubes are filled with the phase separated solution and scanned by a laser beam to detect the surface between condensed phase and solute phase. Preferably, the capillary tubes are centrifuged prior scanning to coalesce the condensed phase into one phase.

Thus, in one embodiment, the device comprises a laser light source, a dichroic mirror, a pinhole or a slit, a stage, an objective, a z-drive (a motorized focus drive), and capillary tubes. The light source is directed to a high N.A objective using the dichroic mirror located behind the pinhole or the slit.

The means for calculating a concentration ($c_{con}$) of the condensed phase of the polymer and a concentration ($c_{dil}$) of the dilute phase of the polymer in said system is preferably a computer which is connected to a controller and a detector, wherein the computer is configured to receive signals detected from the detector and wherein the computer is configured to calculate a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}}.$$

**Description of Figures**

**Figure** 1: Concentration series in defined volumes allow for determination of $c_{con}$ and $c_{dil}$. a, Schematic of the binodal of a phase diagram with dilute branch $c_{out}$ and condensed branch $c_{con}$ concentrations connected via a tie line. b, Schematic of the experimental protocol and setup design using a titration of $c_{tot}$ in water-in-oil emulsions mounted on a temperature-controlled device. c, The total concentration of protein $c_{tot}$ used in the volume $V_{tot}$ of an emulsion droplet separates into the dilute phase with $c_{dil}$ and $V_{out}$ and the condensed phase with $c_{con}$ and $V_{in}$. d, Example images of PGL-3 condensates in emulsions mounted on the device in b. The brightfield channel is used to determine the volume of the emulsions droplets while the fluorescent channel is used to determine the condensed phase volume (scale bar 100 $\mu$m). e, Zoom on segmentation of emulsion droplet and condensate of white square region in d (scale bar 10 $\mu$m). f, Using mass and volume conservation, the volume fraction of the condensed phase (**$V_{con}$ / $V_{tot}$**) describes a linear form with the slope $(c_{con}-c_{dil})^{-1}$ and an offset of $c_{dil}/(c_{con-dil})$, allowing to derive both $c_{dil}$ and $c_{con}$ via a linear regression against $c_{tot}$. g, equation 1 and linear regression of the ratio ***$V_{in}$/$V_{tot}$*** and the total polymer concentration, $c_{tot}$. h, calibration of image analysis pipeline with fluorescent polystyrene beads of known size.

**Figure 2:** Salt and temperature dependence of phase separated FUS and PGL-3 protein. a, False colour experimental images of PGL-3 + 5% PGL-3::GFP for various temperatures and concentrations (binodal line is a guide to the eye). b, Temperature vs. protein concentration phase diagrams of dilute ($c_{dil}$) and condensed ($c_{con}$) branch of FUS::GFP for various salt concentration. Derived from linear regression to $c_{tot}$ concentration series for each salt concentration and temperature (error bars are SD). c, Temperature vs. protein concentration phase diagrams for PGL-3 spiked with 5% PGL-3::GFP. d, e, Data of b and c presented as salt concentration vs. protein concentration phase diagrams for FUS and PGL-3 respectively. f, Comparison of the dilute branch concentrations $c_{dil}$ of FUS and PGL-3 as surfaces in a salt concentration, temperature space.

**Figure** 3: Reversible temperature quenches in and out of the two-phase region for FUS::GFP. a, Dilute branch binodal ($c_{dil}$) for FUS::GFP at different salt concentrations. Solid dots demark the condensation temperature $T_{cond}$ for the protein concentrations used at specific salt concentrations that allow to cross the binodal with a temperature quench. b, Timeseries of temperature quenches from 35°C to 10°C and back to 35°C. Upper panel shows experimental data for the volume fraction and measured temperature curves for four different salt and protein concentrations of FUS::GFP (10 s resolution). Lower panel shows example projections of 3D image stacks at specific time points (also indicated by the red dots on the temperature curve). Yellow circles indicate remaining condensates/aggregates for low salt concentrations after the temperature quench.

**Figure 4:** Comparison of variations of the inventive method (inPhase) with standard bulk measurements for PGL-3. a, Overview of $c_{tot}$ titration series of projections of 3D image stacks for PGL-3 + 5% PGL-3::GFP in 386 well-plates. b, Comparison of dilute branch concentrations $c_{dil}$ for inPhase using fluorescence (fluo) and brightfield (BF) in emulsions and the well-plate approach versus bulk measurements via Pierson BCA (BCA) and 280 nm UV absorption measurements in a nanoDrop (nDrop). c, Comparison of condensed branch concentrations $c_{con}$ for inPhase and bulk approaches. Dashed line indicates the value for the emulsion fluorescence-based evaluation as a standard.

**Figure 5:** Effect of temperature and salt on the partition factor and comparison to quantitative fluorescence. a, b, Partition factor $PF_c$ ($c_{con}$ / $c_{dil}$) derived via inventive method (inPhase) for various temperatures and salt concentrations for FUS::GFP and PGL-3 + 5% PGL-3::GFP respectively (y-axis logarithmic). c, Partition factor as a function of the amount of labelled PGL-3 protein fraction. While $PF_c$ is derived via inPhase $PF_I$ is the partition factor calculated via fluorescence intensities in the dilute and condensed phase of the same sample. d, Partition factor $PF_I$ of individual

condensates as a function of their volume for FUS::GFP and GFP and PGL-3 + 5% PGL-3::GFP respectively. Upper and lower panels contain the same data with the lower panels depicted with a logarithmic x-axis (dashed lines indicate PFc as derived via inPhase method for the same data-set). e, Relative partition factor of inPhase $PF_c$ divided by quantitative intensity-based $PF_I$ for various salt concentrations and temperatures for FUS and PGL-3.

**Figure 6:** shows an exemplary setup for the determination of the height of a condensed phase layer within a container (no need for fluorescent labels). This representation uses the reflection of light at the interface to determine the thickness of a layer.

**Figure 7:** shows the time-dependent changes of condensed and solute concentration for two exemplary proteins.

**Figure 8:** shows *in vivo* volume fraction over total protein concentration data from P granules *in C. elegans* embryos.

**Figure 9:** shows comparison of the effect of untreated control (DMSO) and Lipoamide on the phase diagram and partition factor of FUS.

**Figure 10:** shows a schematic drawing of the device according to the invention.

**Figure 11:** shows a diagram of the intensity of reflected light in dependency of the sample depth.

**Figure 12:** RNA to protein ratio dependence of phase separated FUS. a, molar RNA to protein ratio vs. total concentration (RNA + protein) phase diagrams of dilute ($c_{dil}$) and condensed ($c_{con}$) branch of FUS::GFP for various RNA to protein ratios. Derived from linear regression to $c_{tot}$ concentration series for each RNA to protein ratio; b, volume fraction vs. total concentration (RNA + protein) at a constant molar RNA to protein ratio of 0.01. Experiments were carried out as described in Example 5 with Poly(A) RNA as RNA and FUS-GFP as protein.

**Examples**

**General Method**

The invention relates to the study of protein solutions that undergo phase separation into a dense and a dilute phase. It allows to determine the protein concentrations of both the dense and the dilute phase. It relies on measuring the volume fraction of the condensed phase in the total reaction for a titration series of total protein concentration $c_{tot}$.

$$\frac{V_{con}}{V_{tot}} = \frac{1}{c_{con}-c_{dil}} \cdot c_{tot} - \frac{c_{dil}}{c_{con}-c_{dil}}$$

linear form

$$\left(\frac{1}{c_{con}-c_{dil}} \text{ and } \frac{c_{dil}}{c_{con}-c_{dil}} \text{ are constants}\right)$$

**Protocol 1 (water-in-oil emulsions):**

1. Measure the protein concentration of the stock solutions. The stock solutions can be unlabelled, a mix of labelled and unlabelled or fully labelled protein. Solutions containing only unlabelled protein will be detected using brightfield (or phase-contrast, DIC, etc.) while the mixes that contain labelled protein can be also detected with fluorescence means.
2. Make a titration series of the protein solution.
3. Trigger phase separation of the protein samples. Phase separation can be triggered for example by a change in salt, pH or temperature.
4. Immediately after triggering phase separation, encapsulate in a water-in-oil system (e.g. PicoSurf oil (Sphere Fluidics, UK)).
5. Centrifugation after encapsulation is optional but helps in the segmentation of the condensates. This is because

the centrifugation coarsens the condensates.

6. Load the titration series into chambers for microscopy imaging. A custom-built temperature-controlled stage for mounting was used, thereby allowing performing temperature dependent sweep of the binodal.

7. Acquire images of the emulsion droplets and the encapsulated condensates. This allows to determine the volume of the emulsion droplet as well as the condensate. Nowthe ratio condensate over emulsion droplet volume which is the volume fraction can be determined. In case of fluorescently labelled proteins the determination of the condensate volume can be complemented by fluorescence microscopy.

8. Perform the image and segmentation analysis and fit the measured volume-fractions to a linear regression over the protein concentrations.

9. Determine the $c_{con}$ and $c_{dil}$ using the linear form derived from mass- and volume-conservation equations.

10. Repeat under different conditions (temperature, pH, salt, small molecule concentration) to generate a phase diagram.

**Protocol 2 (well-plate):**

Step 1.-3. are the same as in **Protocol 1.**

4. Immediately after triggering phase separation, pipette the samples in the well plate.

5. Centrifugation is recommended to coarsen the condensates. Otherwise longer waiting for coarsening or large z-stacks have to be acquired.

6. Acquire images of the condensates in the well. The sample volume in a well is known and thus the volume fraction is the result of the segmented condensate volume divided by the sample volume.

Continue with steps 8.-10. in **Protocol 1.**

**Example 1:**

**PGL-3 protein purification:**

PGL-3 was purified from insect cells according to Saha et al. (Cell 166, 1572-1584.e16 (2016)) from SF9-ESF cells were infected with baculovirus containing the PGL-3-GFP-6HIS protein under the polyhedrin promoter. Cells were harvested after 3 days of infection by centrifugation at 500 × g for 10 min and then resuspended in lysis buffer (25 mM HEPES 7.25, 300 mM KCl, 10 mM imidazole, 1 mM DTT, 1 protease inhibitor). Cells were lysed by passing the cells 2 times through the LM20 microfluidizer at 15 000 psi. The lysate was then centrifuged at 20 000 rpm for 45 min at 15 °C. The lysate was loaded in a pre-equilibrated Ni-NTA column with lysis buffer at 3 mL/min. The Ni-NTA column was rinsed with 10 C.V of wash buffer (25 mM HEPES 7.25, 300 mM KCl, 20 mM imidazole, 1 mM DTT, 1) and the protein was eluted in 1.5 mL fractions with elution buffer (25 mM HEPES 7.25, 300 mM KCl, 250 mM imidazole, 1 mM DTT). After elution the GFP tagged was cleaved to produce untagged PGL-3. The cleavage was performed using a TEV protease overnight at 4 °C. PGL-3 and PGL-3-GFP proteins (SEQ ID No: 2) were diluted with Dilution buffer (25 mM Tris pH 8.0, 1 mM DTT) to reach 50 mM KCl before loading the protein in an anion exchange HiTrapQ HP 5 mL column. The HiTrap column was previously equilibrated first with HiTrapQ elution buffer (25 mM Tris pH 8.0, 50 mM KCl, 1 mM DTT) and then with HiTrapQ binding buffer (25 mM Tris pH 8.0, 1 M KCl, 1 mM DTT). The column was mounted in a Äkta Pure FPLC system. After the sample was loaded the column was washed with HiTrapQ binding buffer. The sample was finally eluted with a linear gradient from 0 to 55% of HiTrapQ elution buffer (25 mM Tris pH 8.0, 1 M KCl 1 mM DTT) for 25 C.V. Finally, a 100% HiTrap elution buffer step was performed for 5 C.V. The pooled fractions were then loaded in a HiLoad 16/60 Superdex 200 size exclusion chromatography column that was previously equilibrated with Superdex buffer (25 mM HEPES 7.25, 300 mM KCl, 1 mM DTT). After size exclusion, the final samples were collected.

**FUS protein purification:**

Unlabeled FUS purified from a baculovirus construct containing N-HIS-MBP-FUS-TEV-SNAP. SF9-ESF cells were harvested after three days of infection by centrifugation at 500 × g for 10 min. The cell pellet was resuspended using 50 mL of lysis buffer (50 mM Tris pH 7.4, 500 mM KCl, 5% glycerol, 10 mM imidazole, 1mM PMSF, 1X protease inhibitor) for every 50 mL of cultured cells. The cells were lysed by passing them 2 times through the LM20 microfluidizer at 15 000 psi. The lysate was then centrifuged at 20 000 rpm for 45 min at 15 °C. The supernatant was collected and loaded into a Ni-NTA column that was previously equilibrated with lysis buffer. After loading the sample, the column was washed for 10 C.V. with Ni-NTA was buffer (50 mM Tris pH 7.4, 500 mM KCl, 5% glycerol, 20 mM imidazole). The protein was then eluted with Ni-NTA elution buffer (50 mM Tris pH 7.4, 500 mM KCl, 5% glycerol, 300 mM imidazole).

The collected fractions where then loaded into a MBPTrap HP column preequilibrated with Ni-NTA elution buffer. The MBP column was washed for 10 C.V with MBP wash buffer (50 mM Tris pH 7.4, 500 mM KCl, 5% glycerol). After washing, the sample was eluted with MBP elution buffer (50 mM Tris pH 7.4, 500 mM KCl, 5% glycerol, 500 mM arginine, 20 mM maltose). The protein was diluted to a concentration of less than 15 μM using MBP elution buffer. 3C and TEV proteases were then added to cleave the MBP and SNAP tags from the FUS construct. The cleavage reactions were incubated overnight at 18 °C. Finally, the protein was loaded in a SepFast GF-HS-L 26 × 600 mm gel filtration to remove the cleaved MBP and SNAP tags and exchange the buffer. The SepFast column was previously equilibrated in storage buffer (50 mM HEPES pH 7.25, 750 mM KCl, 5% glycerol, 1 mM DTT). The sample was concentrated to a final concentration of ~15 μL using 30 kDa Amicon centrifuge filters. FUS-GFP (SEQ ID No: 1) was purified as previously described (Wang, J. et al. "A Molecular Grammar Governing the Driving Forces for Phase Separation of Prion-like RNA Binding Proteins", Cell, 2018, 174, 688-699.e16).

**Protocol *in vitro* protein handling:**

Protein solutions were prepared from stocks that are frozen at -80°C. The protein stock solutions were cleared from potential aggregates using centrifugal filters (UFC30HV00, Merck, Germany). The protein concentrations were determined via extinction coefficient measurements at 280 nm (NanoDrop, Thermo Fisher Scientific, USA). Next, the desired label fraction, in the main experiments 5% GFP labeled protein, was prepared. In order to keep the protein from phase separating to early the titration of the final protein concentrations was done at salt concentrations of the protein stock (300mM KCl for PGL-3 and 750 mM KCl for FUS; both 25mM HEPES and 1 mM DTT at 7.4pH). Only in the final step the salt concentration was dropped to the desired value, using a no salt buffer (25 mM HEPES and 1 mM DTT at 7.4 pH), and the solution was immediately encapsulated into the water-in-oil solution. Encapsulation was performed using twice the amount of PicoSurf oil (2% (w/w) in Novec 7500, Sphere Fluidics, UK) compared to the protein solution (usually 5 μL). After adding the oil on top of the protein solution in standard 100μl microcentrifuge tubes the solution is agitated using a 10μl pipette until the desired size distribution of water-in-oil solutions is reached. This solution can be used directly for imaging. Alternatively, the coarsening of the phase separated condensates can be speed up by a mild centrifugation step of the water-in-oil emulsions (100-1000 g for 3 min). This will lead to predominantly one single condensate of condensed phase per emulsion droplet.

**Sample loading and imaging procedure:**

As a sample holder a custom-made temperature stage based on Peltier elements connected to a sapphire microscope slide (25×75 mm) was used. This allows for fast and precise temperature changes both below and above room temperature. Between the sapphire slide and a standard cover slide (22×22 mm, 170μm, Menzel, Germany) lanes of Paraflim (Bernis, USA) stripes were sandwiched. The sandwich is connected by heating the stage to at least 55°C which will connect the Parafilm to the top and bottom by applying soft pressure on the cover slide. This allows to load multiple samples in a separated fashion in one measurement. Emulsion solutions are loaded into the individual lanes by pipetting the turbid upper layer in the microcentrifuge tubes. Each lane is sealed using addition curing silicone (Picodent twinsil speed, Picodent, Germany) to ensure no evaporation and movement of the water-in-oil emulsions upon temperature changes. Sample imaging was performed via CellSens software (Olympus, Japan) on an Olympus IX83 microscope connected to a Yokogawa W1 SoRa spinning-disc system (Yokogawa, Japan) and a Hamamatsu Orca Flash v3 sCMOS camera (Hamamatsu, Japan) using a 40x air objective (UPLXAPO, 0.95NA, Olympus, Japan). For each sample, parafilm lane, a multi-tile brightfield image and a corresponding multi-tile 3-dimensional fluorescence stack was recorded using a piezo stage (Mad city labs, USA).

**Image analysis:**

Images were processed by a custom-written pipeline using MATLAB software (The Mathworks, USA). The brightfield images of each lane were used to detect the contours of the water-in-oil emulsion droplets. The location as well as the volume of each emulsion droplet was determined considering the height of approximately 100 μm of the Parafilm chambers. Corresponding positions in the fluorescence image stack are further analyzed after removing emulsion droplets that are intersected by the margins of the imaged tiles as well as overlapping emulsions. For each emulsion droplet its condensed phase is determined by image registration and parameters like total condensed phase and intensity values are extracted. In brief, after basic image filtering, an intensity gradient approach was used to find a good estimate for the outline of the condensates. To get a good mask for the solute phase, first the masks of the condensed phase were dilated and then removed them from the mask of the emulsion droplet. A possible influence of intensity values close to the margins of condensates can thus be minimized.

**Example 2. Time dependent behavior of the condensates**

It has been observed, that the material properties of condensates change over time. The material properties of a condensate are relevant for their function. This is especially interesting for the ageing of condensates that are linked to neurodegenerative diseases (e.g. hardening of FUS condensates related to ALS). However, it remains challenging to measure material properties on such small scales. Here, it is shown that changes in the phase (diagram) can be tracked over time using the inventive approach (Figure 7).

1. Prepare the sample according to protocol 1 or 2 (e.g., a total protein concentration titration at phase separating conditions, at the desired external conditions (temperature, salt concentration, pH, pressure, buffer composition, crowding agent)).
2. Perform time-dependent measurement of the volume fraction of the condensed phase (e.g. via fluorescence microscopy based imaging using a labeled protein, or brightfield imaging using unlabeled protein, or any of the other techniques outlined herein).
3. Use linear regression to fit the volume fraction versus total concentrations data for each time-point and apply our formula to get to a time-dependent $c_{in}(t)$ and $c_{out}(t)$.
4. A time-dependent plot of either $c_{in}$ or $c_{out}$ (or any derivative value of the two, e.g. the ratio) allows assessing the ageing of the protein condensed phase of interest.

**Example 3. Effect of small molecule drugs**

To demonstrate the possibilities of drug screening, a small molecule that has been indicated to affect the phase separation of FUS, a protein related to ALS has been used. It has so far been difficult to accurately the extent of the effect because of a lack of quantitative measures (Figure 9).

Here the effect of Lipoamide on the phase behavior of FUS is shown, where a reduced amount of conformational changes at higher temperatures was observed. This is indicated by the earlier bent of the condensed phase branch of the binodal for the untreated control (DMSO). This can also be seen in the partition factor $c_{in}$ / $c_{out}$.

1. Prepare the sample according to protocol 1 or 2 (e.g., a total protein concentration titration at phase separating conditions).
2. Add small molecule drug of interest to the final protein mix before encapsulation for the emulsion approach or before/after adding protein mix to the well-plates (depending on intention to measure the effect of the drug during formation of the condensates (before) or on the already formed condensates (after)).
3. Use linear regression to fit the volume fraction versus total concentrations data for each drug concentration and apply our formula to get to a dose-dependent $c_{in}$ and $c_{out}$ compared to a control (e.g., DMSO control).
4. This allows preparing an IC50 plot for the effect of the small molecule drug.

Example 4. Phase diagrams ***in vivo***

In general, the method of the present invention can be used for any phase separating system. This is also true for *in vivo* settings of phase separating proteins. The natural or induced fluctuations in the total protein concentration of cells are equivalent to our protein concentration titration series. Thus, the inventive method can be applied to calculate $c_{out}$ and $c_{in}$. As previously discussed, the volume fraction of the phase and the total concentration of protein (e.g. via GFP labelled protein) is determined. This is shown exemplary for *C. elegans* embryos and the protein PGL-1 that is constitutive for P granules (Figure 8).

1. Determine the volume fraction of the condensed phase of a protein inside a cell (e.g., using a fluorescently labelled protein (live imaging) or fixed cells with immunofluorescence).
2. Determine the total concentration of the protein of interest within the given cell. For example, by using the mean intensity of the protein in the cell and relating it to a known standard (e.g., fluorophore concentration titration, quantitative immunoblots see (Fritsch et al. PNAS, 2021, 118(37), e2102772118), mass spectrometry see (Saha et al Cell 166, 1572-1584.e16 (2016))).
3. Use the intrinsic variability of the total concentration between cells to plot the volume fraction over total protein concentration.
4.a. In addition, RNAi can be used to decrease the total concentration of a given protein in vivo in a controlled way.
4.b In addition, inducible gene expression (e.g. doxycycline based) can increase the total concertation of a given protein in vivo in a controlled way. The gene can be integrated in the genome or transfected in a plasmid.
5. Use linear regression to fit the volume fraction versus total concentrations data for and apply equation 1 to get

$c_{out}$ and an apparent $c_{in}$. Given that condensates *in vivo* are mostly multicomponent systems the apparent $c_{in}$ is assuming the condensate is only made up of the measured protein.

**Example 5: Effect of RNA on protein phase separation**

FUS-GFP containing samples were prepared as described in Example 1. Phase separation was triggered by simultaneously lowering the salt concentration of the sample and adding the Poly(A) RNA in an amount to maintain a fixed molar ratio of Poly(A):FUS-GFP. The volume fraction of the condensed phase was determined as described in Example 1 and $c_{dil}$ and $c_{con}$, were determined, wherein $c_{con}$ represents an apparent concentration containing both Poly(A) (SEQ ID No: 3) and FUS-GFP concentration. The concentration of the single components of the sample can be resolved by the knowledge of the molar ratio of RNA to protein in the condensed phase.

## Claims

**1.** A method for determining a concentration ($c_{con}$) of a polymer in a condensed phase and a concentration ($c_{dil}$) of the polymer in a dilute phase in a system comprising:

A) determining a total volume ($V_{tot}$) of the system and a total concentration ($c_{tot}$) of the polymer;
B) triggering phase separation of the polymer into a condensed phase and a dilute phase in said system;
C) determining a volume fraction ($V_{con}$ / $V_{tot}$) of the condensed phase of the polymer in said system;
D) determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

E) changing at least one condition of said system and repeating the steps A) to D).

**2.** The method according to claim 1, wherein the polymer is a protein, or a polynucleic acid, preferably RNA, DNA, a mixture of a protein and RNA or a mixture of a protein and DNA and/or wherein the system is an *in vitro* system and is selected from a solution, an emulsion, or cells.

**3.** The method according to claim 1 or 2, wherein in step B),
the phase separation of the polymer in said system is triggered by changing: the concentration of a component in the system selected from a salt, a crowding agent, or a buffer; the pH value; the pressure; or the temperature of the system.

**4.** The method according to any one of claims 1 to 3, wherein when the polymer is not labelled with a fluorophore or a fluorescent protein, in step A) the total volume ($V_{tot}$) of said system and the total concentration ($c_{tot}$) of the polymer are determined by means of bright-field, dark-field, phase-contrast, holographic, polarization, or differential interference correlation (DIC) microscopy, or light-scattering based approaches.

**5.** The method according to any one of claims 1 to 4, wherein when the polymer is labelled with a fluorophore or a fluorescent protein, in the step A) the total volume ($V_{tot}$) of said system and the total concentration ($c_{tot}$) of the polymer are determined by means of fluorescence microscopy.

**6.** The method according to any one of claims 1 to 5, wherein step C) comprises:

C1a) encapsulating a portion of the system in an emulsion system;
C2a) determining a volume ($V_{em}$) of one emulsion droplet of the emulsion system, and a condensed volume ($V_{con}$') of the condensed phase of the polymer in said emulsion system;
C3a) determining a volume fraction ($V_{con'}/V_{em}$) of the condensed volume ($V_{con}$') of the condensed phase of the polymer in said emulsion system and the volume ($V_{em}$) of one emulsion droplet of said emulsion system, wherein the measured volume fraction ($V_{con'}/V_{em}$) is identical to the volume fraction ($V_{con}/V_{tot}$) of the condensed volume ($V_{con}$) of the condensed phase of the polymer in the system and the total volume ($V_{tot}$) of the system.

**7.** The method according to claims 6, wherein in the step C2a) a volume ($V_{em}$) of the emulsion system, and a condensed volume ($V_{con}$') of the condensed phase of the polymer in said emulsion system are determined by means of fluorescence microscopy.

**8.** The method according to claim 6 or 7, further comprising after the step C2a):
C2b) adjusting the measured fluorescent image with a predetermined partition factor.

**9.** The method according to any one of claims 1 to 8, further comprising after step B):
B') centrifuging the system.

**10.** The method according to claims 1 to 9, wherein in step E) the at least one condition of said system is selected from a concentration of a component in the system wherein the component is selected from a salt, or a buffer; the pH value; the pressure; a crowding agent; or the temperature of the system, or a combination of the aforementioned conditions.

**11.** An assay method for identifying bioactive compound(s), comprising:

A) determining a total volume ($V_{tot}$) of the system and a total concentration ($c_{tot}$) of a polymer in the system;
A') adding a predetermined amount of test compounds into said system;
B) triggering phase separation of the polymer into a condensed phase and a dilute phase;
C) determining a volume fraction ($V_{con}$ / $V_{tot}$) of the condensed phase of the polymer in said system;
D) determining a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}},$$

F) identifying the bioactive compounds from the test compounds; and a device comprises a plurality of systems.

**12.** The assay method according to claim 11, further comprising after step D):
D') comparing the measured condensed volume ($V_{con}$), the measured condensed concentration ($c_{con}$), and/or the measured volume fraction ($V_{con}/V_{tot}$) of said polymer in the system with a condensed volume ($V_{ref}$), a condensed concentration ($c_{ref}$), and/or a volume fraction ($V_{ref}/V_{tot}$) of the polymer in the presence of a reference molecule in a control system.

**13.** The assay method according to claim 12, wherein the condensed volume ($V_{ref}$), the condensed concentration ($c_{ref}$), and/or the volume fraction ($V_{ref}/V_{tot}$) of the polymer in the presence of a reference molecule is(are) predetermined or measured at the same time.

**14.** The assay method according to claim 12 or 13, wherein the identified bioactive compound(s) increase(s) the measured condensed volume ($V_{con}$), the measured condensed concentration ($c_{con}$), the measured dilute concentration ($c_{dil}$), and/or the measured volume fraction ($V_{con}/V_{tot}$) of the polymer obtained by the step C) and/or D) than the predetermined condensed volume ($V_{ref}$), the measured condensed concentration ($c_{ref}$), and/or the measured volume fraction ($V_{ref}/V_{tot}$) of the polymer of said polymer in the presence of a reference molecule in the control system.

**15.** A device for determining a binodal curve of a polymer in a system comprising:

i) means for measuring a total volume ($V_{tot}$) of the system;
ii) means for measuring a volume fraction ($V_{con}/V_{tot}$) of a condensed phase of the polymer in said system;
iii) means for calculating a concentration ($c_{con}$) of the condensed phase of the polymer and a concentration ($c_{dil}$) of the dilute phase of the polymer in said system by using a following linear form equation:

$$\frac{V_{con}}{V_{tot}} = \frac{1}{c_{con}-c_{dil}} \cdot c_{tot} - \frac{c_{dil}}{c_{con}-c_{dil}}.$$

Figure 1

a

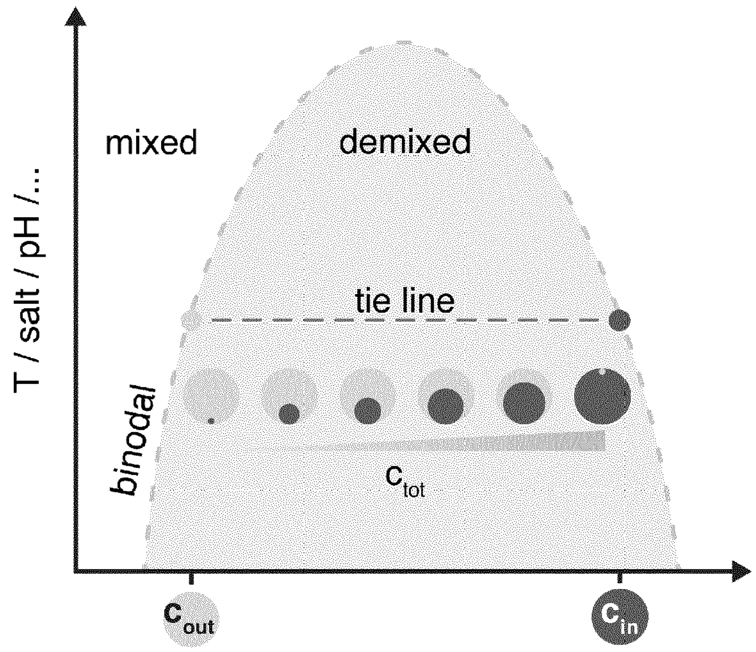
T / salt / pH /...
mixed
demixed
tie line
binodal
$c_{tot}$
$c_{out}$
$c_{in}$

b

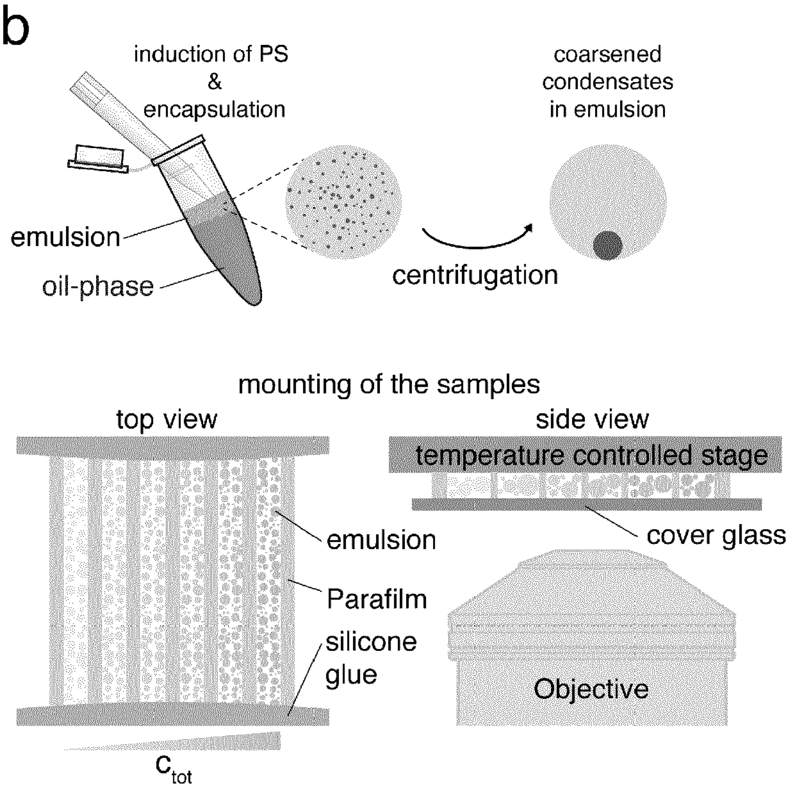
induction of PS
&
encapsulation
coarsened
condensates
in emulsion
emulsion
oil-phase
centrifugation
mounting of the samples
top view
side view
temperature controlled stage
emulsion
cover glass
Parafilm
silicone
glue
Objective
$c_{tot}$

Figure 1 continued

c

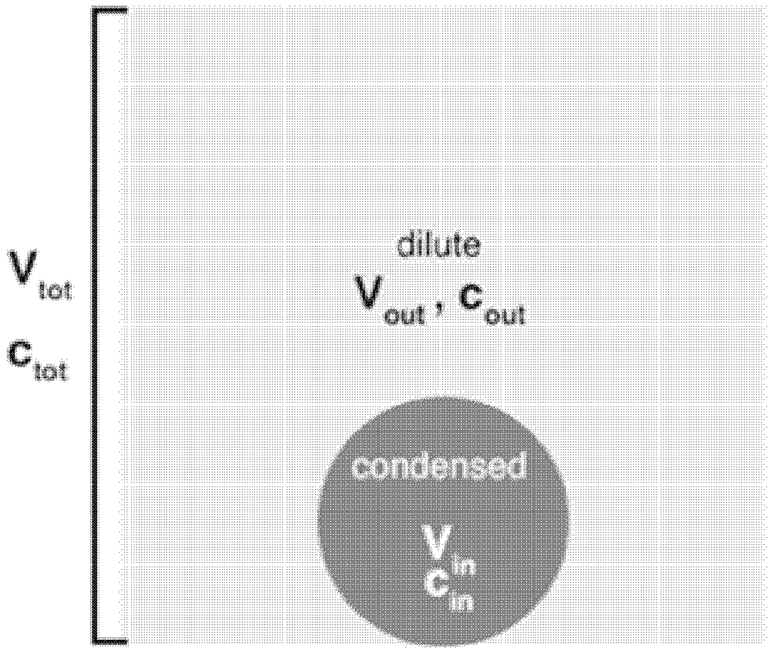


d

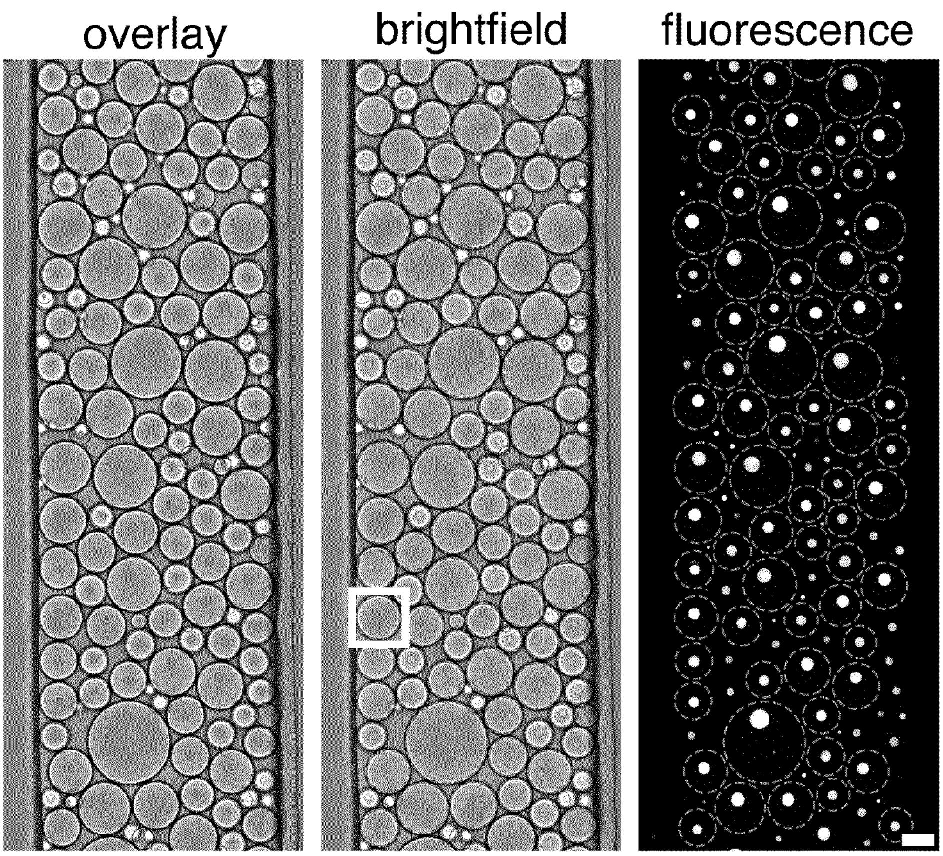

**Figure 1 continued**

e

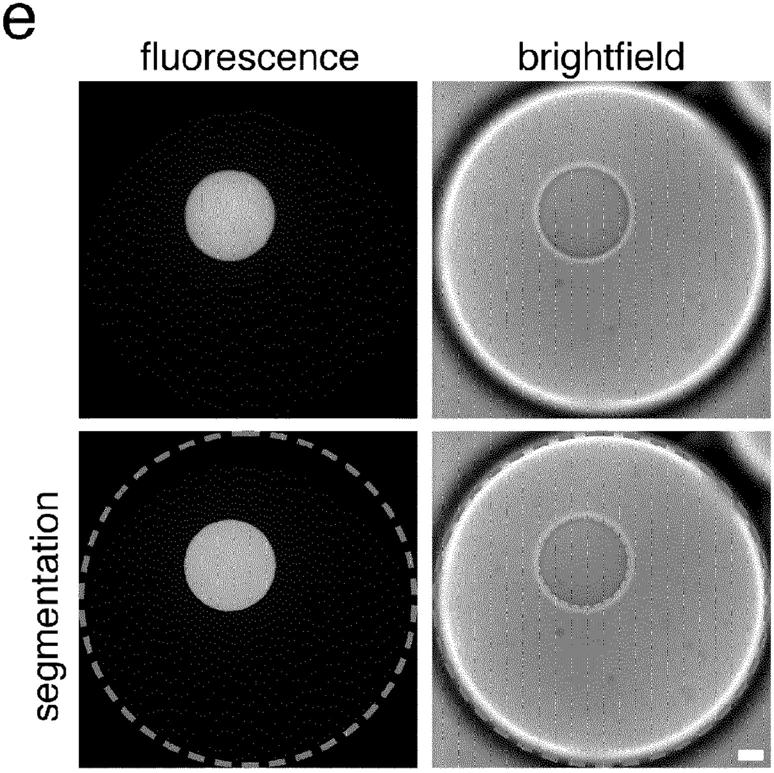


f

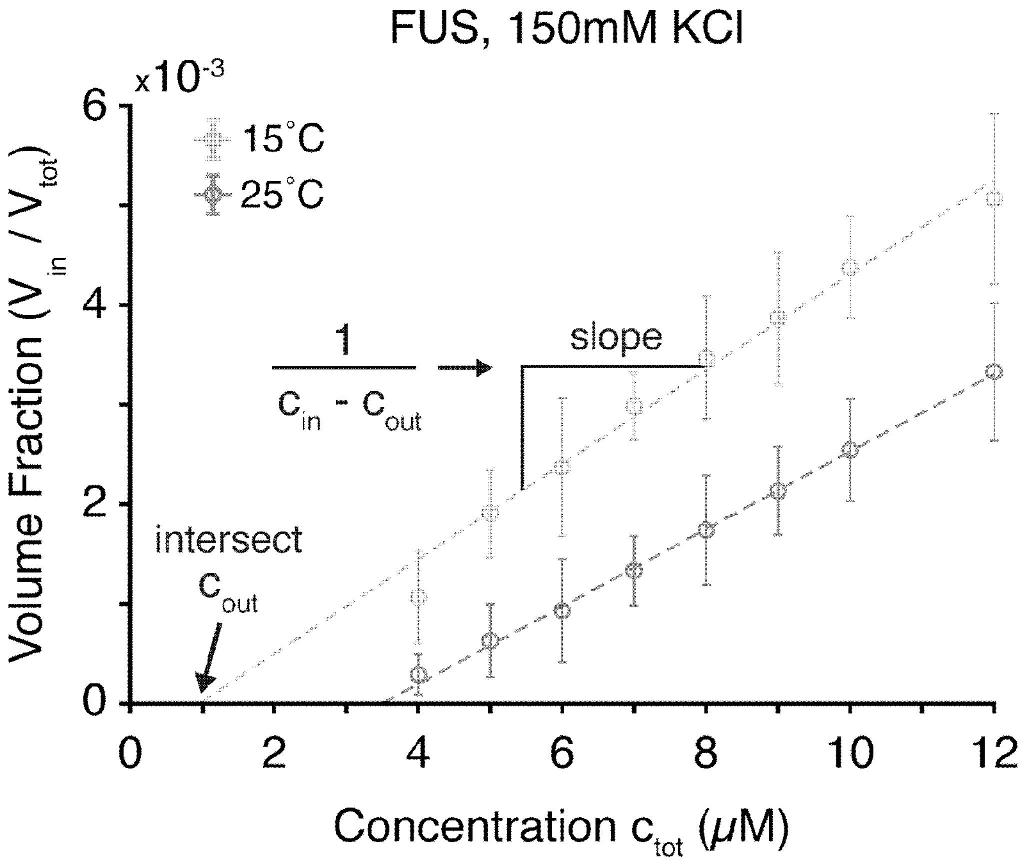

**Figure 1 continued**

# g

$$\frac{V_{con}}{V_{tot}} = \underbrace{\frac{1}{c_{con} - c_{dil}}}_{a\ (slope)} \cdot c_{tot} - \underbrace{\frac{c_{dil}}{c_{con} - c_{dil}}}_{b\ (offset)}$$

$$c_{con} = \frac{1-a}{b} \qquad c_{dil} = -\frac{b}{a}$$

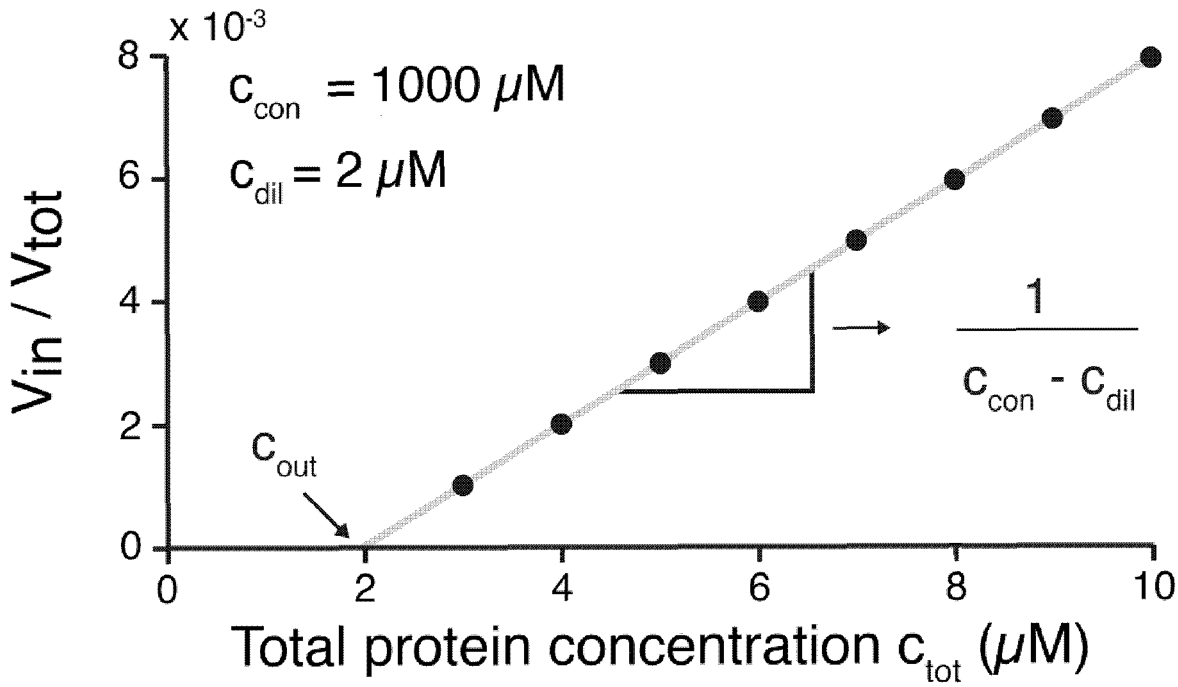

**Figure 1 continued**

**H**

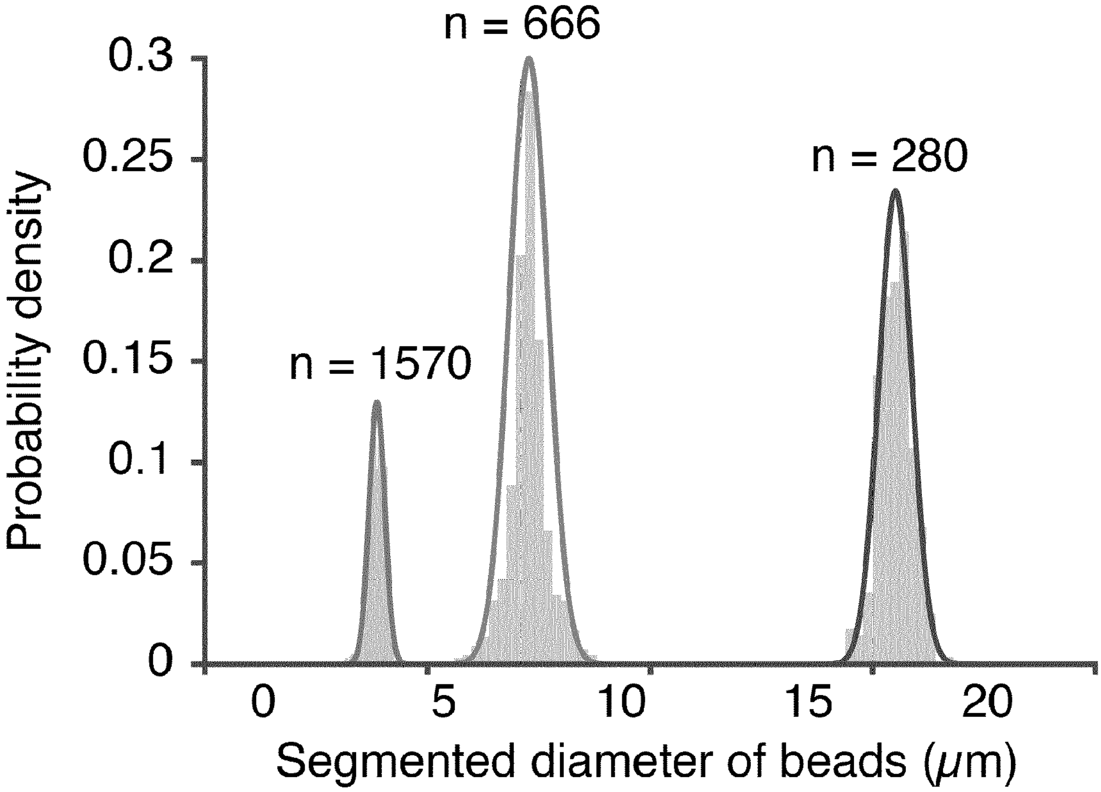


diameter (μm)

| expected | measured |
|---|---|
| 4.2 ± 0.3 | 3.9 ± 0.2 |
| 7.3 ± 0.5 | 7.3 ± 0.4 |
| 15.6 ± 0.7 | 15.5 ± 0.4 |

Figure 2

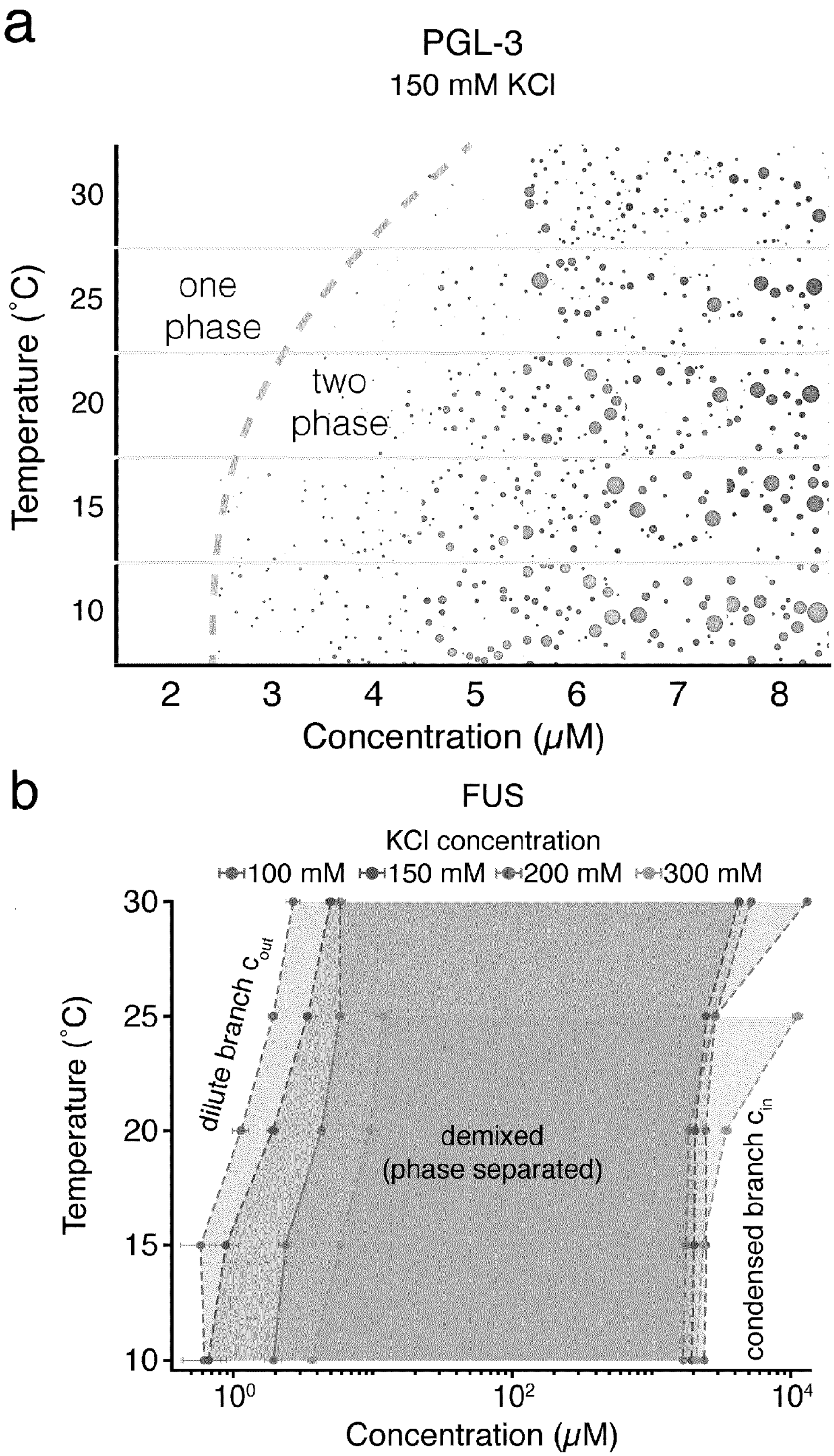
a
PGL-3
150 mM KCl
one phase
two phase
Temperature (°C)
Concentration (μM)
b
FUS
KCl concentration
100 mM
150 mM
200 mM
300 mM
dilute branch $c_{out}$
demixed
(phase separated)
condensed branch $c_{in}$
Temperature (°C)
Concentration (μM)

**Figure 2 continued**

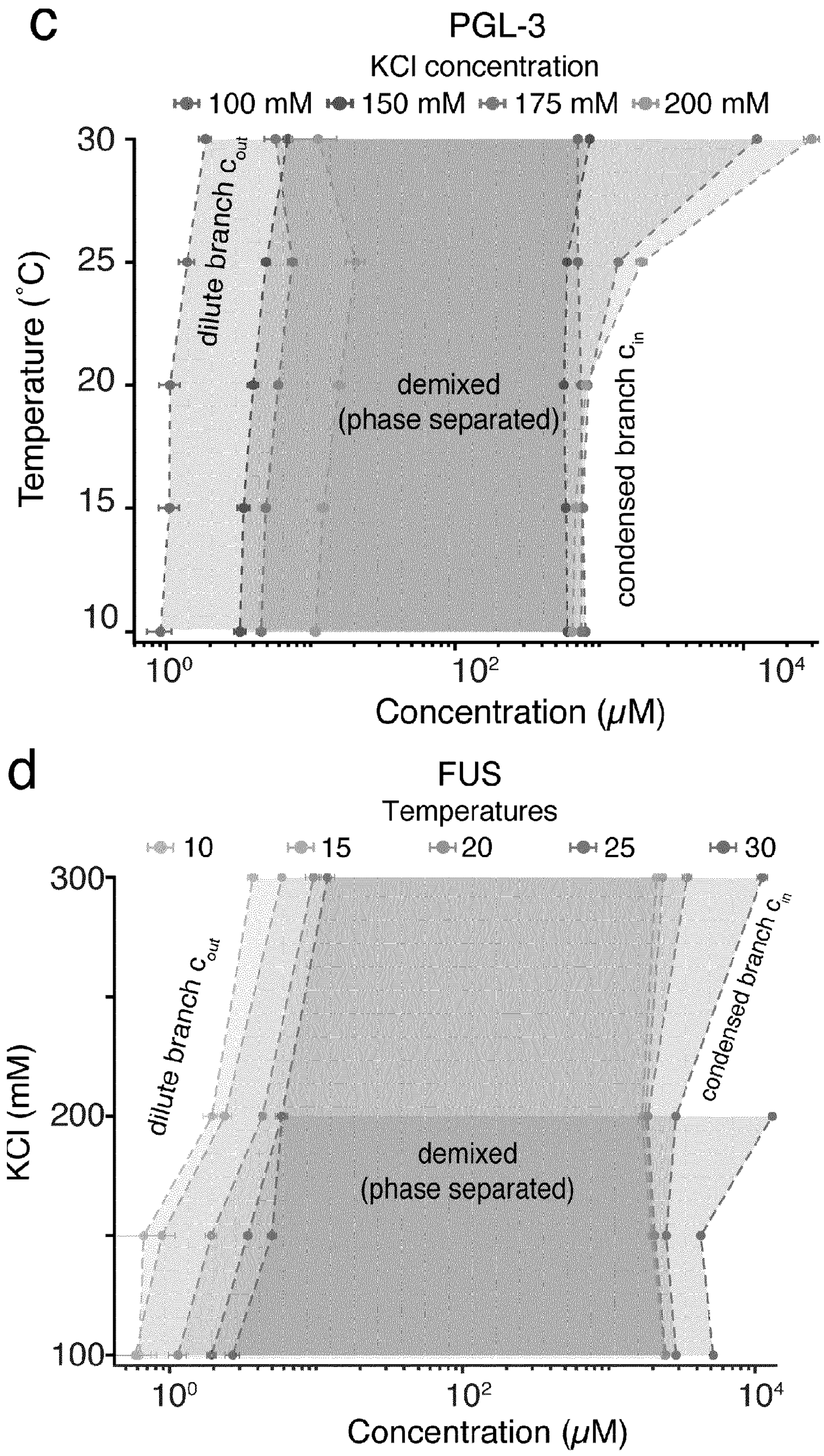

**Figure 2 continued**

e

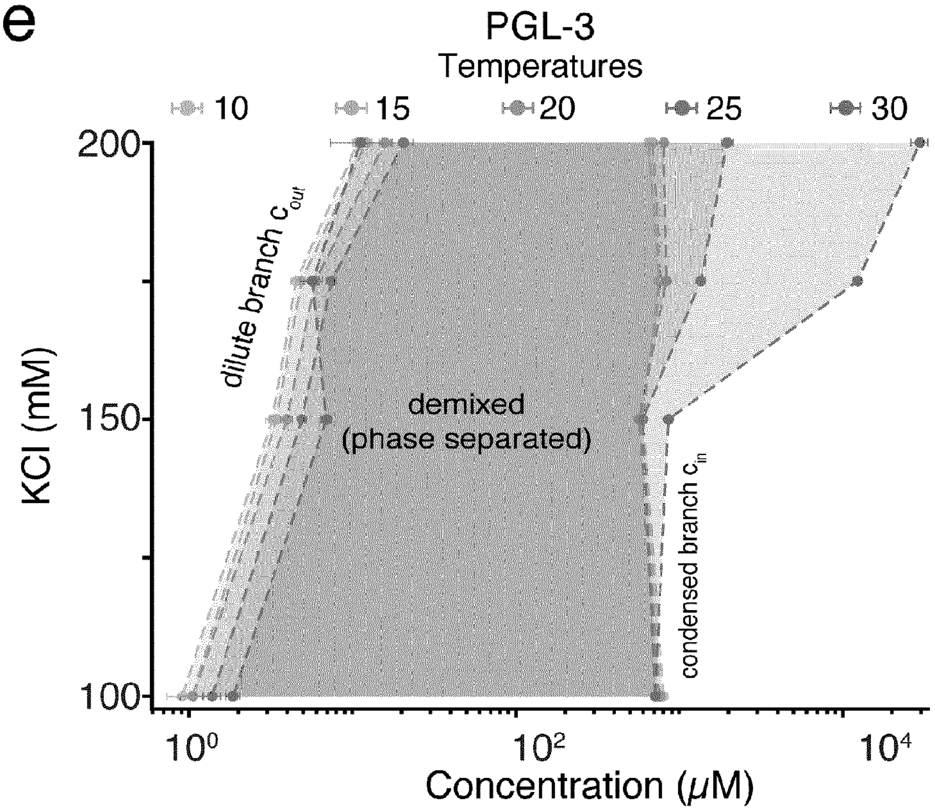


f

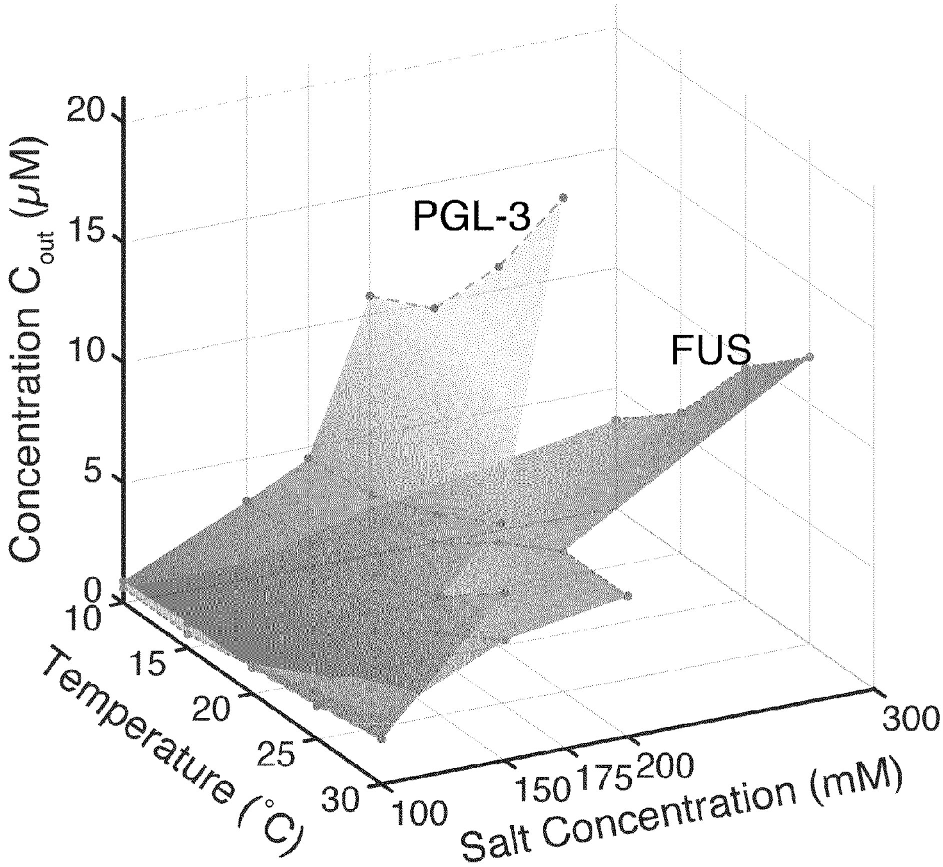

Figure 3

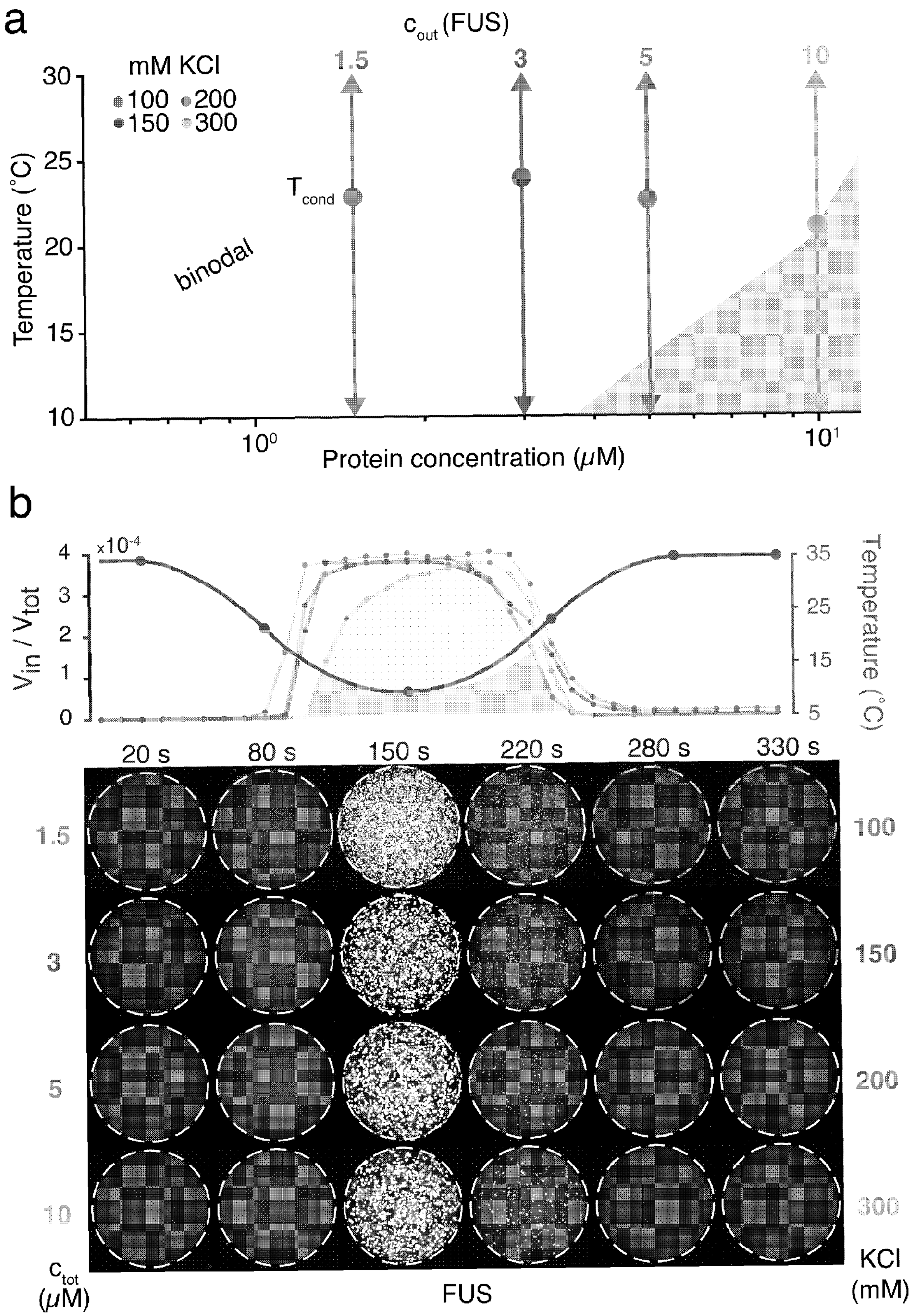
a
c_out (FUS)
mM KCl
100 200
150 300
1.5
3
5
10
30
25
20
15
10
Temperature (°C)
T_cond
binodal
10^0
10^1
Protein concentration (μM)
b
x10^-4
4
3
2
1
0
V_in / V_tot
35
25
15
5
Temperature (°C)
20 s
80 s
150 s
220 s
280 s
330 s
1.5
3
5
10
100
150
200
300
c_tot (μM)
FUS
KCl (mM)

Figure 4

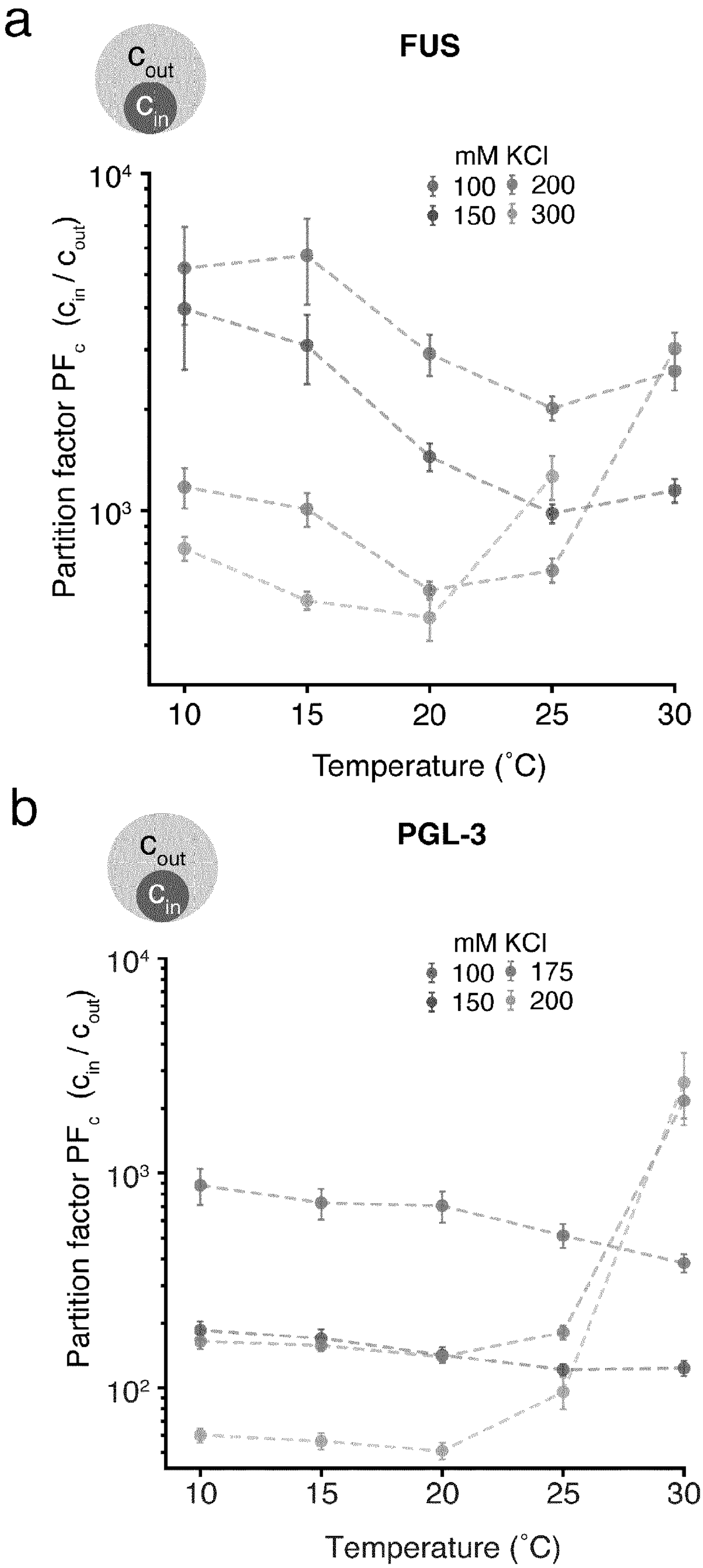
a
FUS
c out
c in
mM KCl
100
200
150
300
Partition factor PFc (cin / cout)
10^4
10^3
10
15
20
25
30
Temperature (˚C)
b
PGL-3
c out
c in
mM KCl
100
175
150
200
Partition factor PFc (cin / cout)
10^4
10^3
10^2
10
15
20
25
30
Temperature (˚C)

**Figure 4 continued**

C

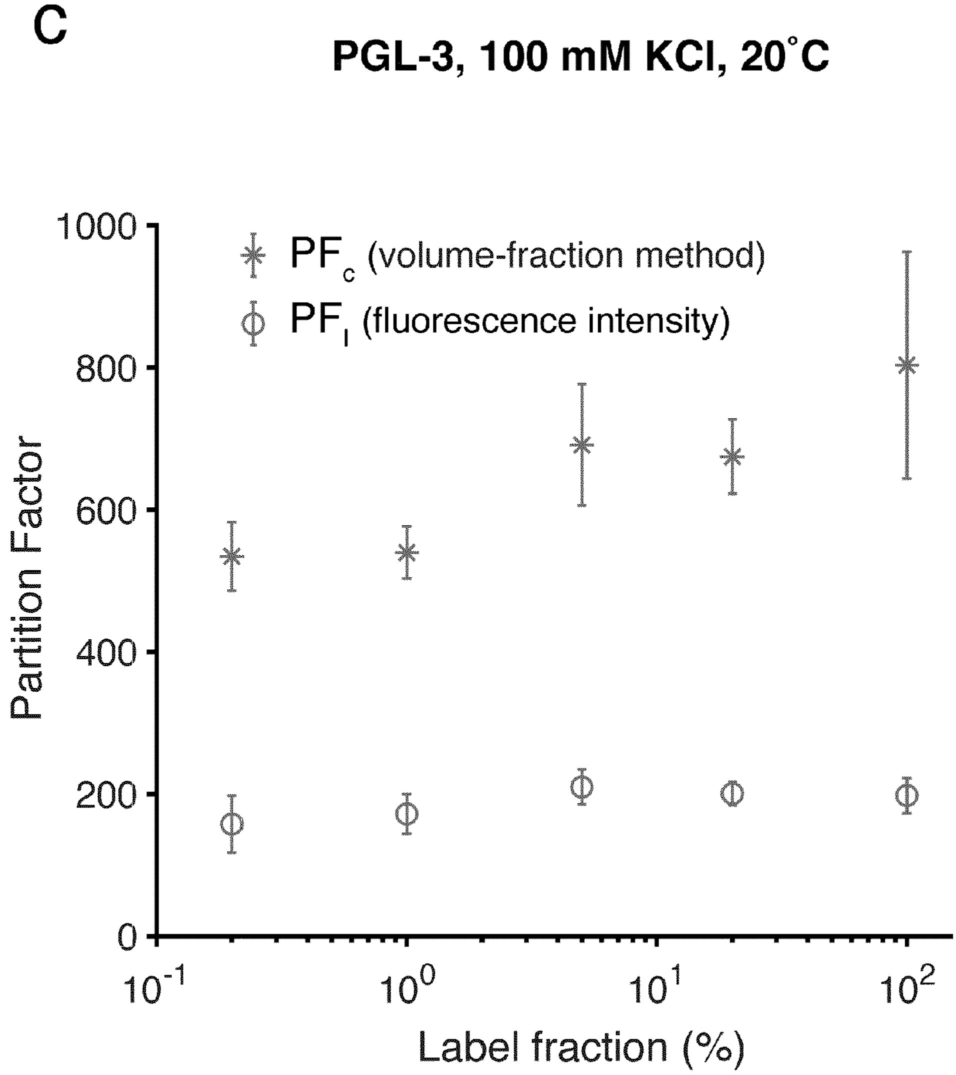

Figure 4 continued

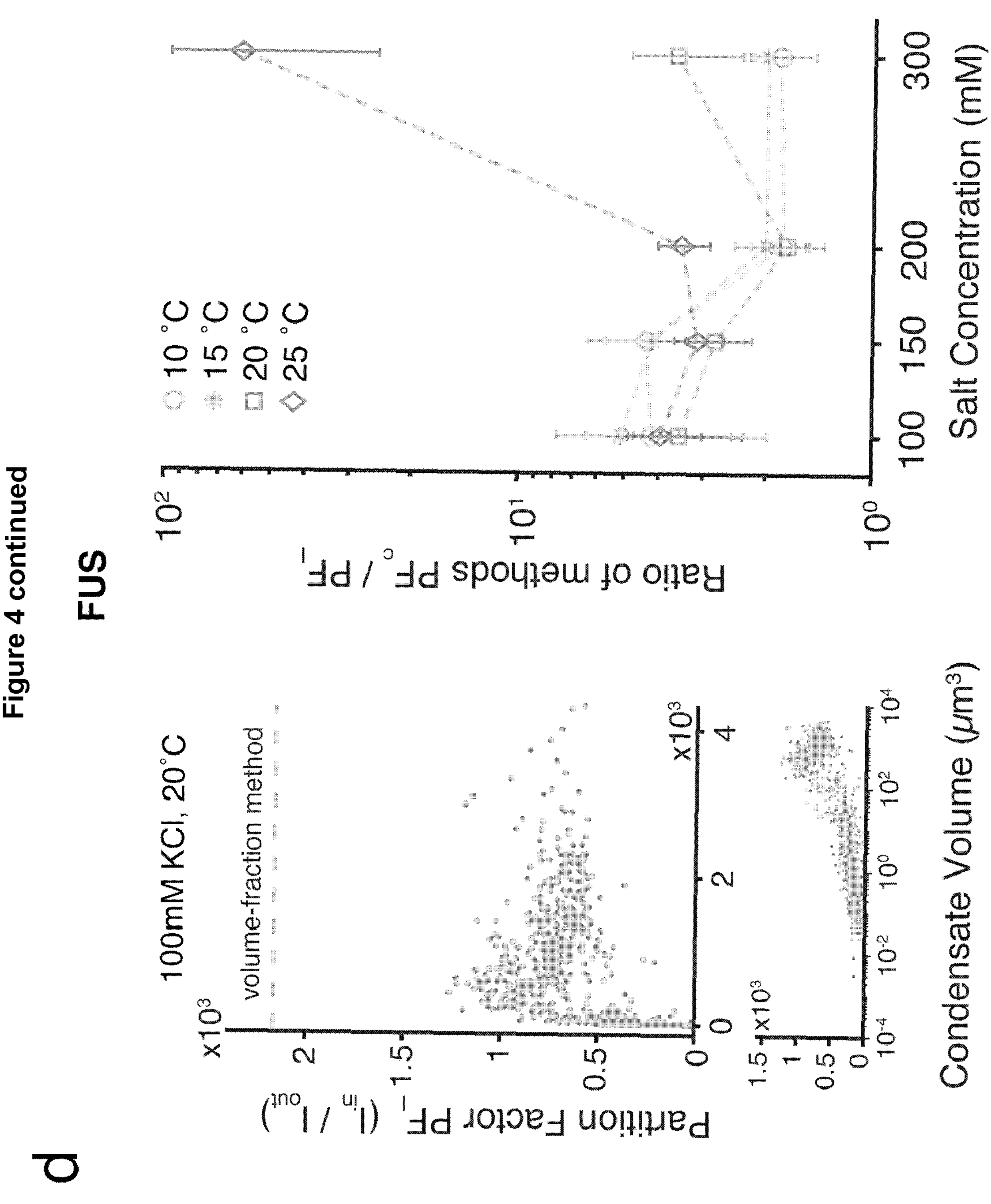

Figure 4 continued

e

**PGL3**

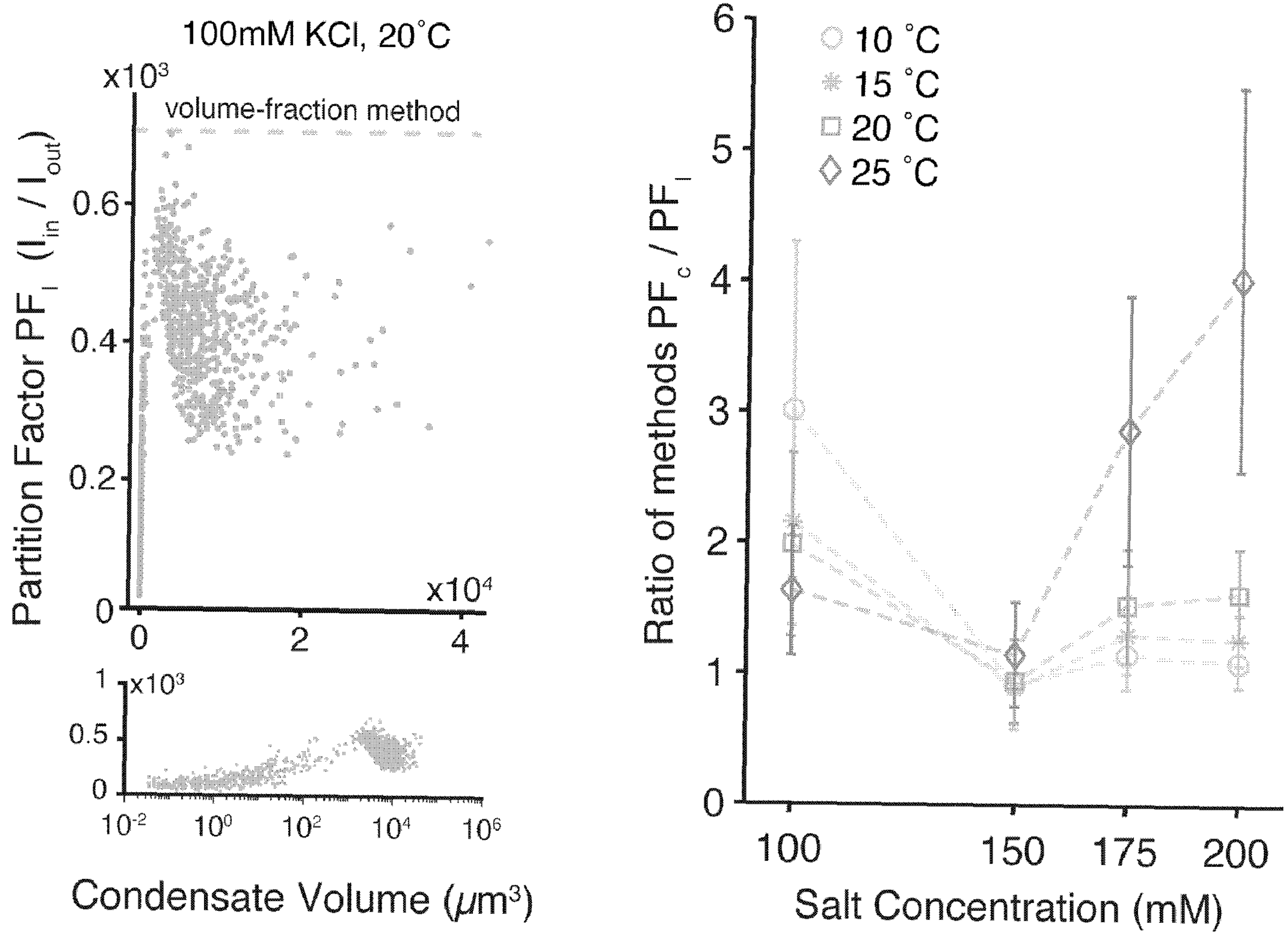

**Figure 5**

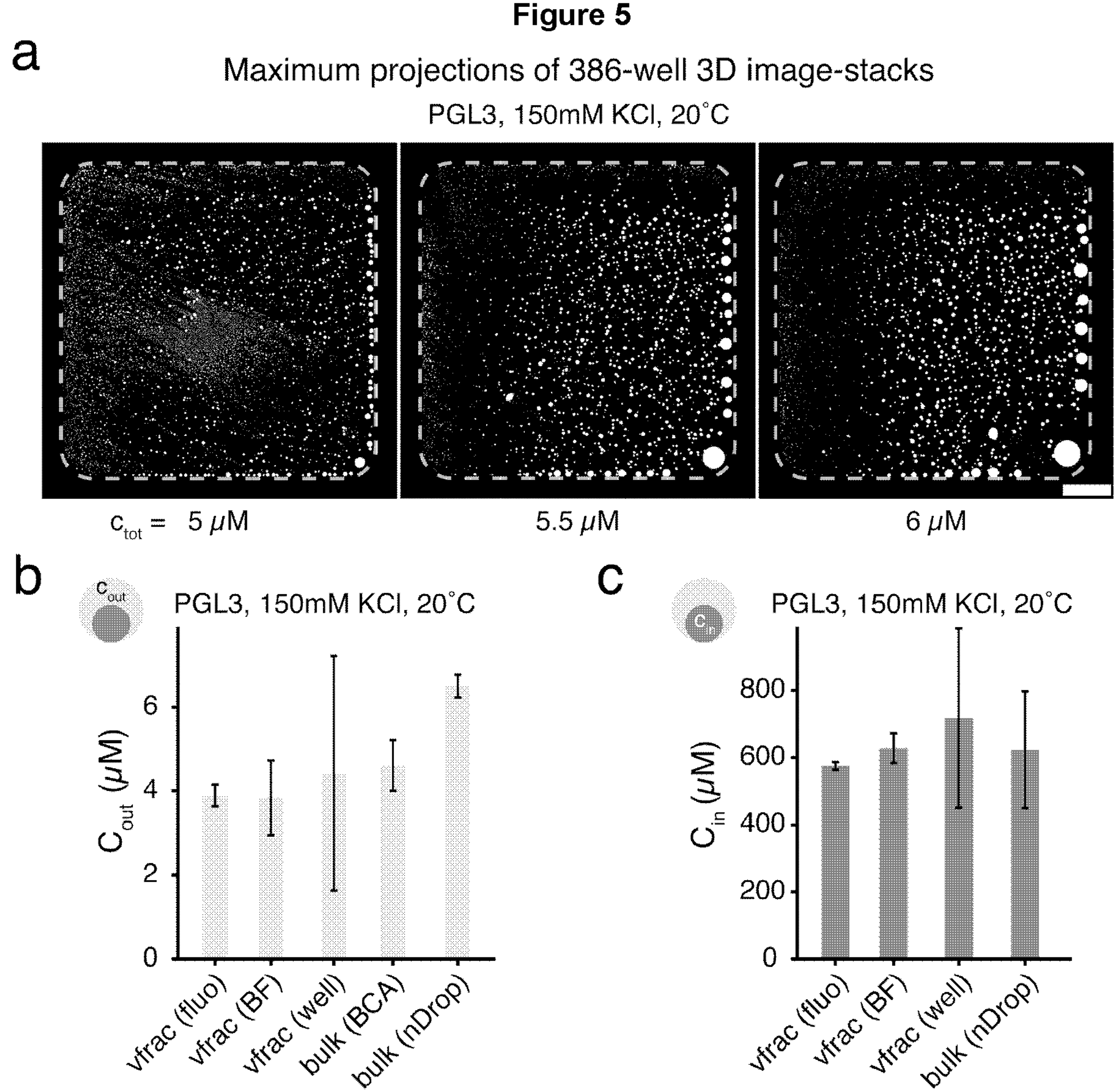
a
Maximum projections of 386-well 3D image-stacks
PGL3, 150mM KCl, 20°C
$c_{tot}$ = 5 μM
5.5 μM
6 μM
b
$c_{out}$
PGL3, 150mM KCl, 20°C
$C_{out}$ (μM)
0
2
4
6
vfrac (fluo)
vfrac (BF)
vfrac (well)
bulk (BCA)
bulk (nDrop)
c
$c_{in}$
PGL3, 150mM KCl, 20°C
$C_{in}$ (μM)
0
200
400
600
800
vfrac (fluo)
vfrac (BF)
vfrac (well)
bulk (nDrop)

Figure 6

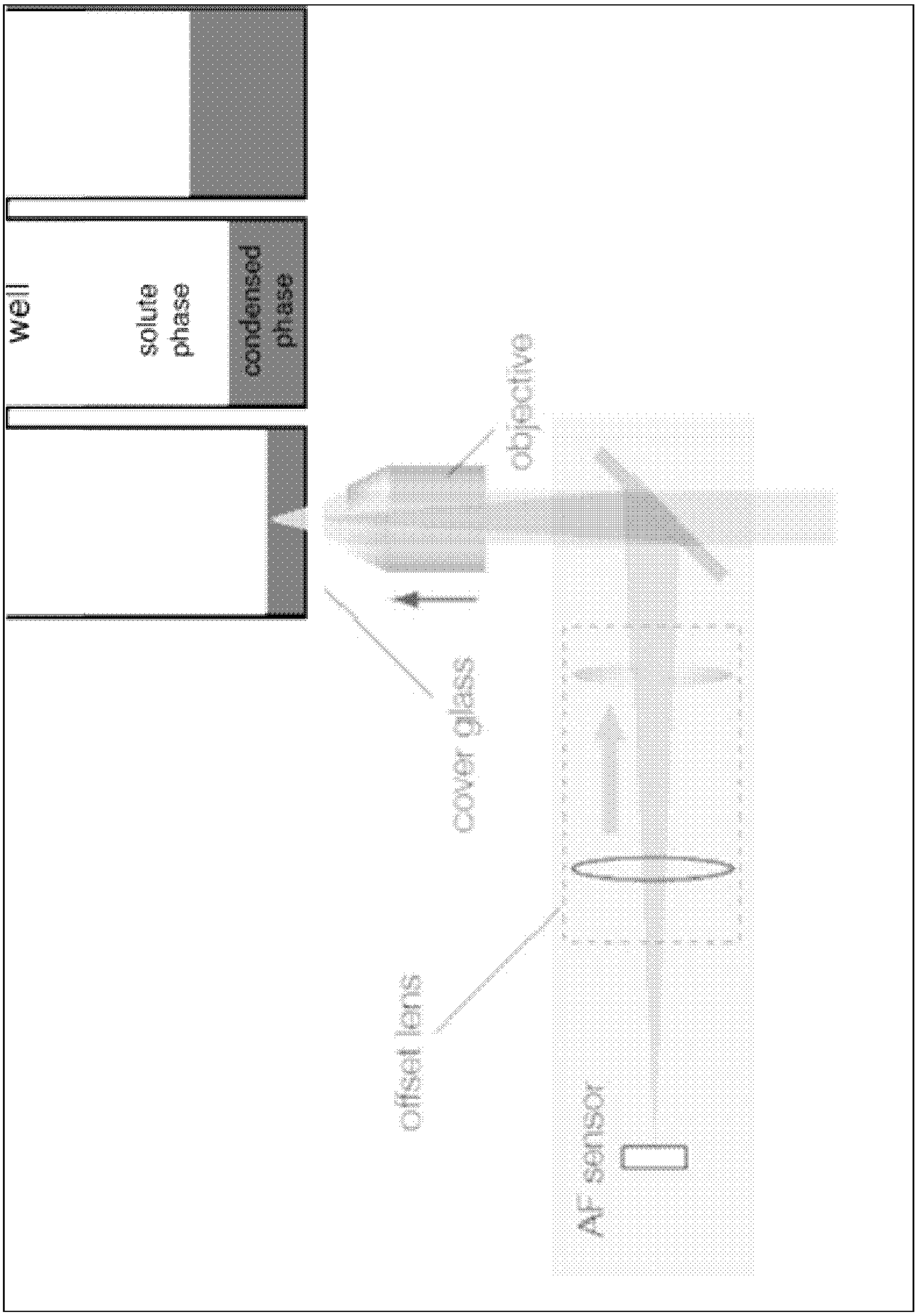
well
solute phase
condensed phase
objective
cover glass
offset lens
AF sensor

Figure 7

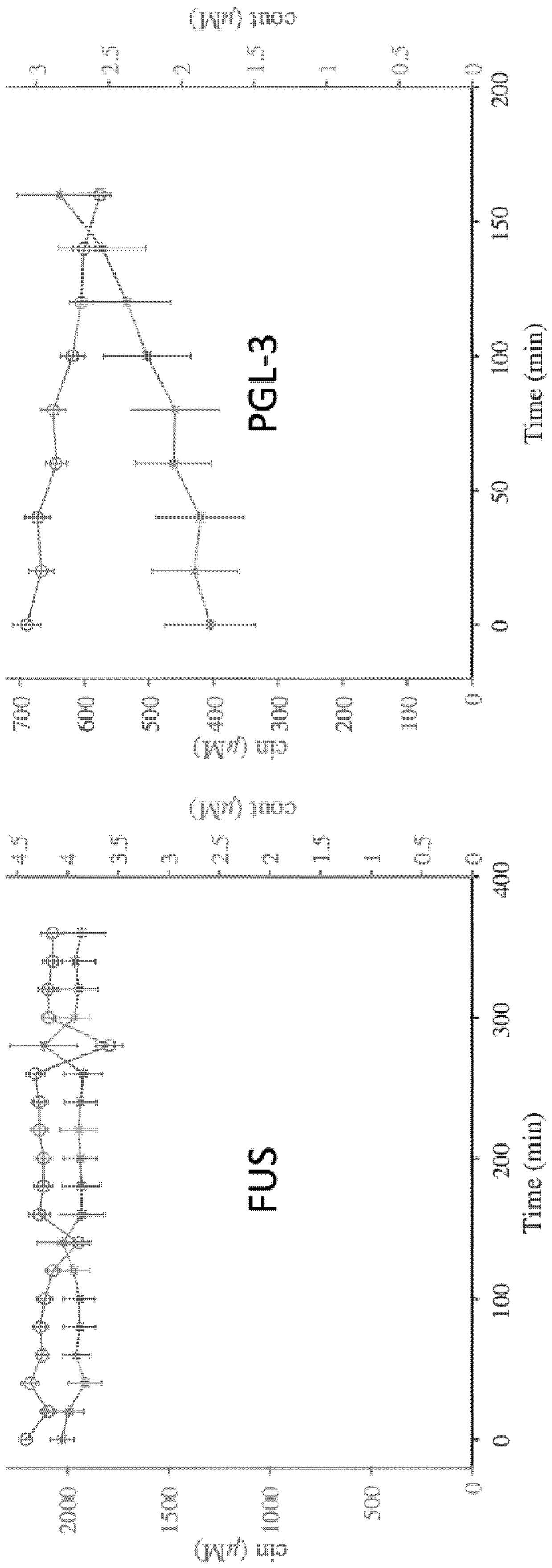
FUS
PGL-3
Time (min)
cin (μM)
cout (μM)
0
50
100
150
200
300
400
500
600
700
1000
1500
2000
0.5
1
1.5
2
2.5
3
3.5
4
4.5

Figure 8

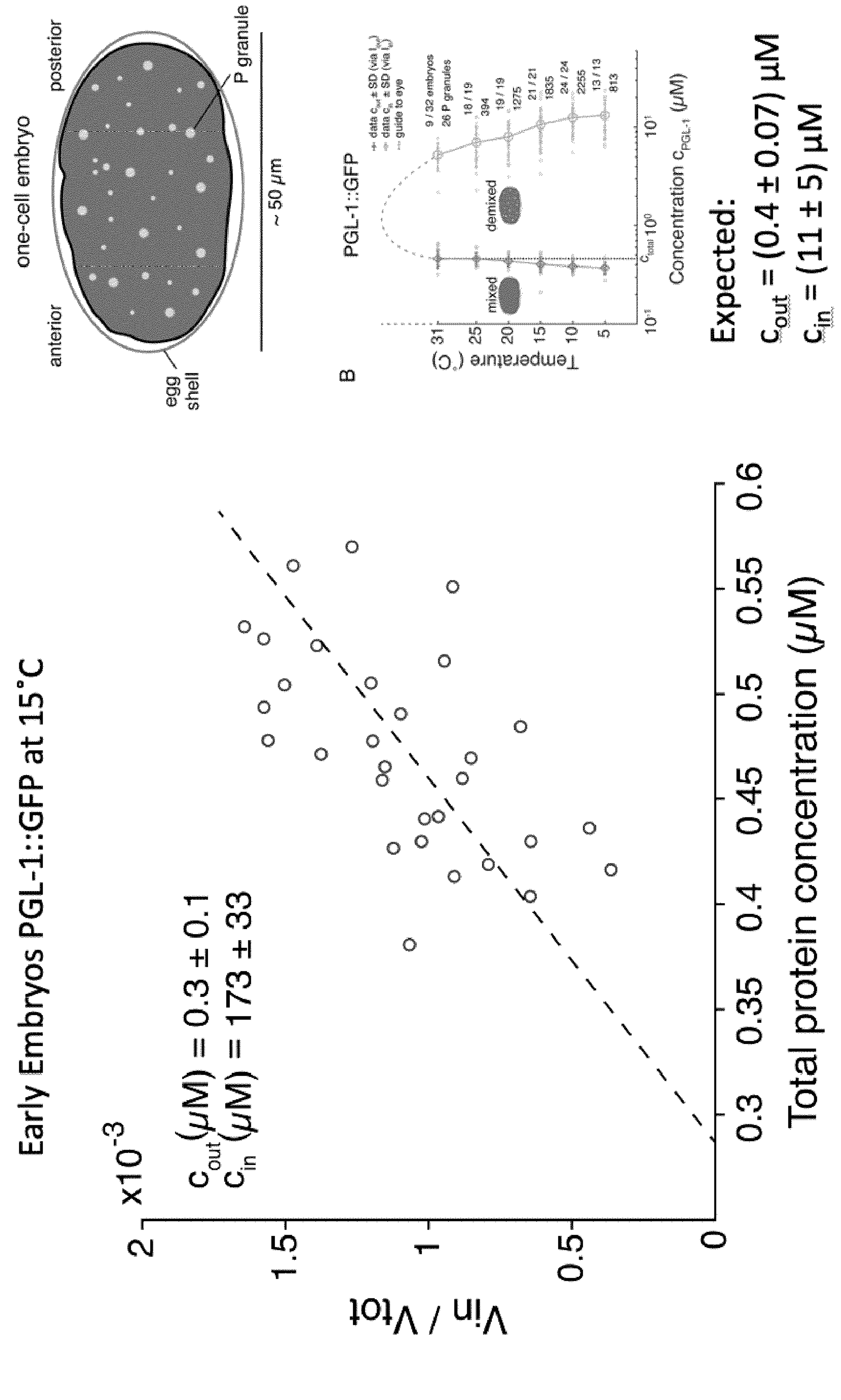
Early Embryos PGL-1::GFP at 15°C
x10$^{-3}$
$c_{out}$ ($\mu$M) = 0.3 ± 0.1
$c_{in}$ ($\mu$M) = 173 ± 33
0
0.5
1
1.5
2
0.3
0.35
0.4
0.45
0.5
0.55
0.6
Total protein concentration ($\mu$M)
$V_{in}$ / $V_{tot}$
one-cell embryo
anterior
posterior
egg shell
P granule
~ 50 $\mu$m
B
PGL-1::GFP
data $c_{out}$ ± SD (via $I_{out}$)
data $c_{in}$ ± SD (via $I_{in}$)
guide to eye
9 / 32 embryos
26 P granules
18 / 19
394
19 / 19
1275
21 / 21
1835
24 / 24
2255
13 / 13
813
mixed
demixed
Temperature (°C)
31
25
20
15
10
5
$10^{-1}$
$c_{total}$ $10^{0}$
$10^{1}$
Concentration $c_{PGL-1}$ ($\mu$M)
Expected:
$c_{out}$ = (0.4 ± 0.07) $\mu$M
$c_{in}$ = (11 ± 5) $\mu$M

Figure 9

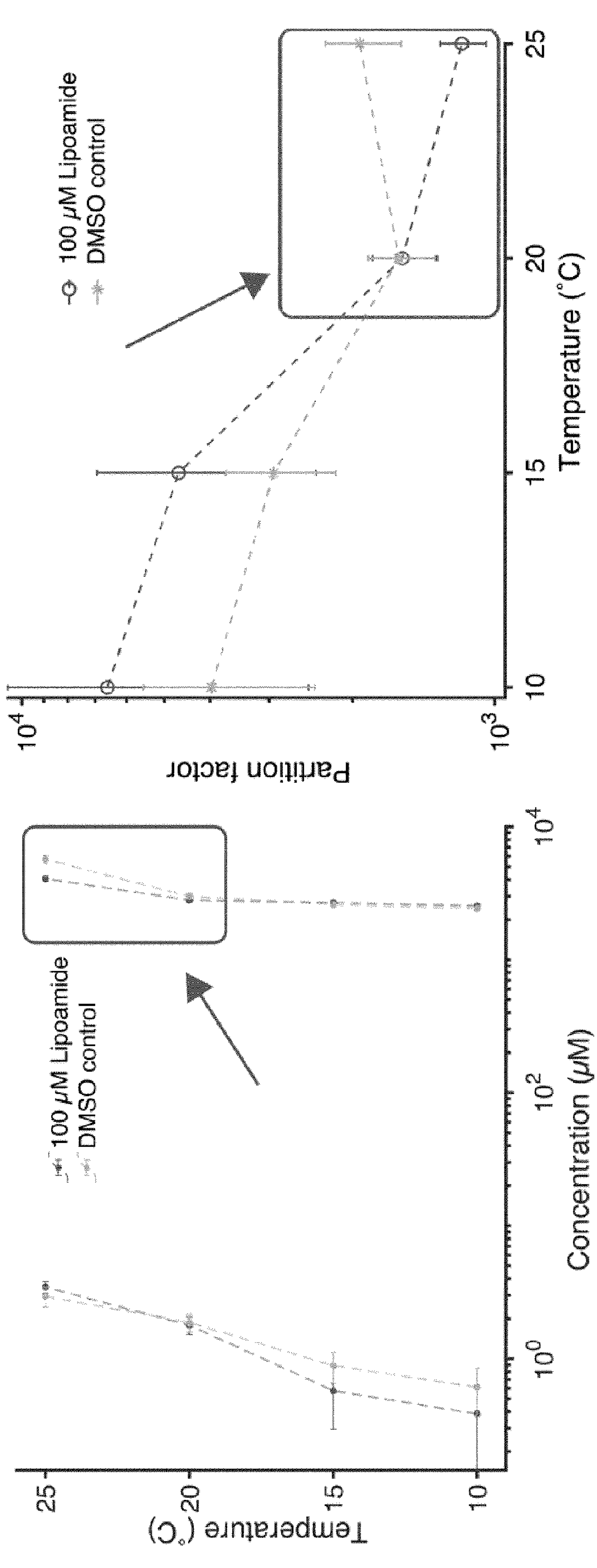
100 μM Lipoamide
DMSO control
Temperature (°C)
Concentration (μM)
Partition factor
Temperature (°C)

**Figure 10**

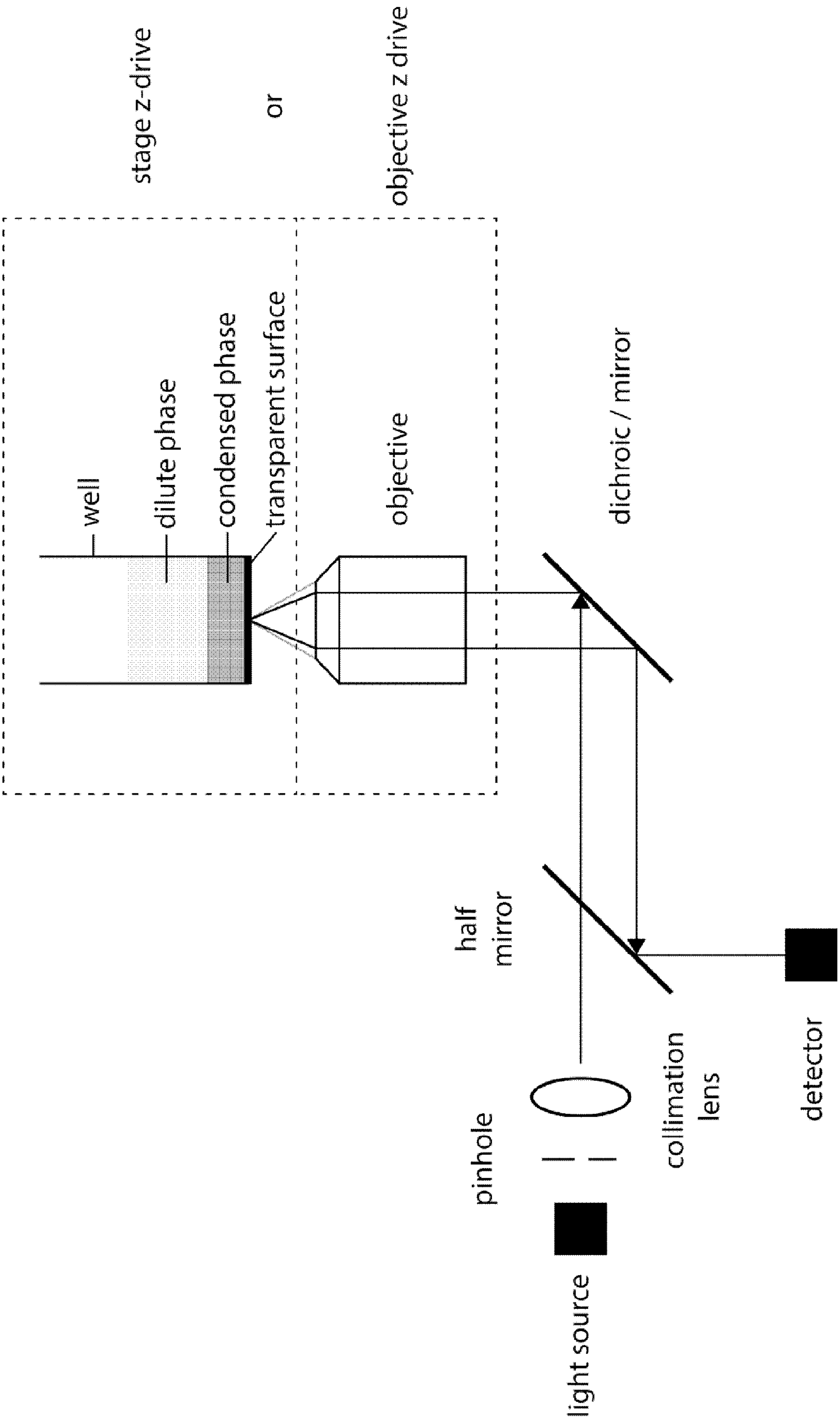
stage z-drive
or
objective z drive
well
dilute phase
condensed phase
transparent surface
objective
dichroic / mirror
half
mirror
pinhole
collimation
lens
detector
light source

**Figure 11**

Intensity of reflected light along sample depth

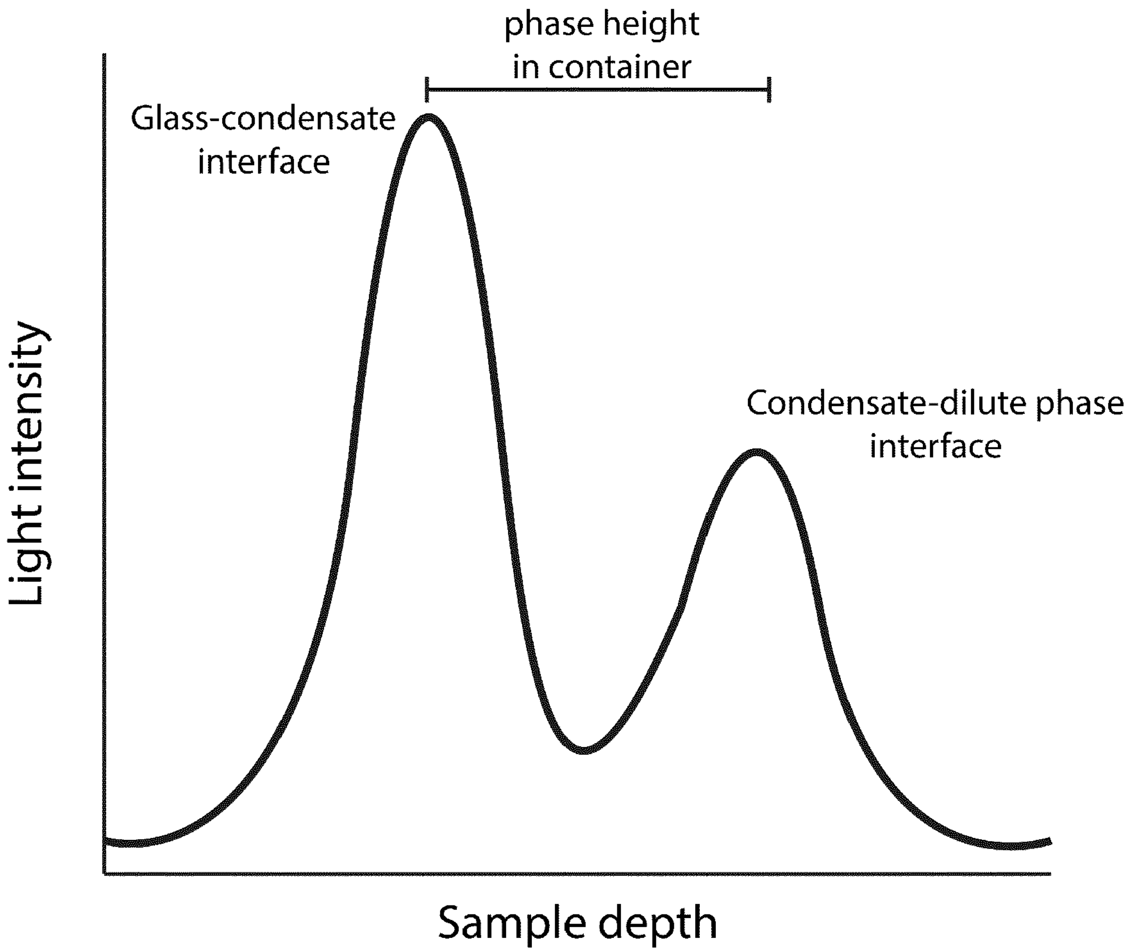

**Figure 12**

A

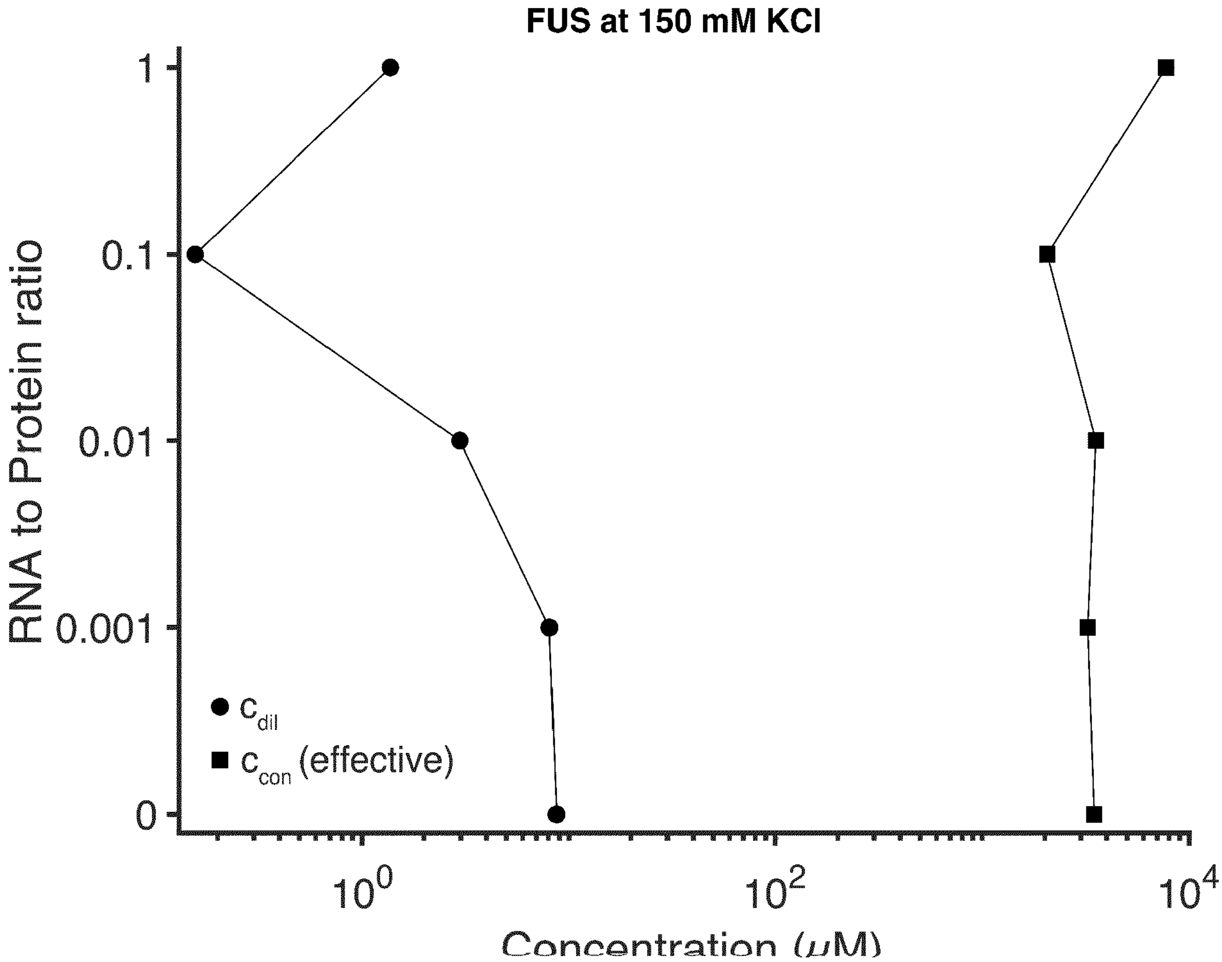
FUS at 150 mM KCl
RNA to Protein ratio
1
0.1
0.01
0.001
0
c dil
c con (effective)
10^0
10^2
10^4
Concentration (μM)

B

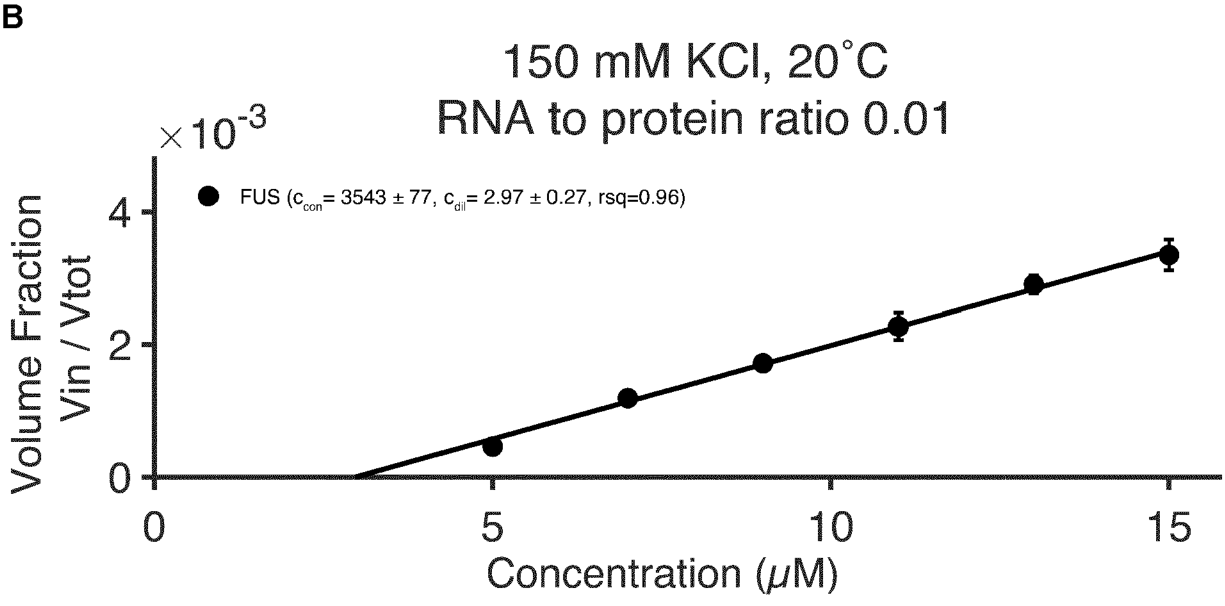
150 mM KCl, 20°C
RNA to protein ratio 0.01
×10^-3
FUS (c con= 3543 ± 77, c dil= 2.97 ± 0.27, rsq=0.96)
Volume Fraction
Vin / Vtot
4
2
0
0
5
10
15
Concentration (μM)

Europäisches Patentamt
European Patent Office
Office européen des brevets

# EUROPEAN SEARCH REPORT

Application Number
EP 22 17 7321

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/350875 A1 (BRANGWYNNE CLIFF [US] ET AL) 11 November 2021 (2021-11-11)<br>* par 0006-0014, 0040-0041, 0074-0077, 100; cl 1-4, 9, 12-25. *<br>----- | 1-14 | INV.<br>G01N33/50<br>G01N33/58 |
| X | DAN BRACHA ET AL: "Mapping local and global liquid-liquid phase behavior in living cells using light-activated multivalent seeds",<br>BIORXIV,<br>16 March 2018 (2018-03-16), pages 1-16, XP055694847,<br>DOI: 10.1101/283655 | 15 | |
| A | * pg 3, last par-pg 4, second par; fig 3. *<br>----- | 1-14 | |
| A | WO 2011/042509 A2 (UNIV STRASBOURG [FR]; CENTRE NAT RECH SCIENT [FR] ET AL.)<br>14 April 2011 (2011-04-14)<br>* the whole document *<br>----- | 1-15 | TECHNICAL FIELDS SEARCHED (IPC) |
| A | US 2022/003661 A1 (YANG TINGLU [US] ET AL)<br>6 January 2022 (2022-01-06)<br>* the whole document *<br>----- | 1-15 | G01N |

The present search report has been drawn up for all claims

| Place of search | Date of completion of the search | Examiner |
|---|---|---|
| Munich | 2 November 2022 | Motrescu-Hateley, E |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 17 7321

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2022

| Patent document cited in search report | Publication date | Patent family member(s) | Publication date |
|---|---|---|---|
| US 2021350875 A1 | 11-11-2021 | CN 113195513 A | 30-07-2021 |
| | | EP 3853242 A1 | 28-07-2021 |
| | | JP 2022501047 A | 06-01-2022 |
| | | US 2021350875 A1 | 11-11-2021 |
| | | WO 2020061251 A1 | 26-03-2020 |
| WO 2011042509 A2 | 14-04-2011 | EP 2485830 A2 | 15-08-2012 |
| | | US 2013072404 A1 | 21-03-2013 |
| | | WO 2011042509 A2 | 14-04-2011 |
| US 2022003661 A1 | 06-01-2022 | US 2022003661 A1 | 06-01-2022 |
| | | WO 2020124096 A2 | 18-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRADY, J. P. et al.** Structural and hydrodynamic properties of an intrinsically disordered region of a germ cell-specific protein on phase separation. *PNAS,* 2017, vol. 114, E8194-E8203
- **MCCALL, P. M. et al.** *Quantitative phase microscopy enables precise and efficient determination of biomolecular condensate composition.,* 2020, vol. 10 (25), 352823
- **BRACHA, D. et al.** Mapping Local and Global Liquid Phase Behavior in Living Cells Using Photo-Oligomerizable Seeds. *Cell,* 2018, vol. 175, 1467-1480
- **FRITSCH, A. W. et al.** Local thermodynamics govern formation and dissolution of Caenorhabditis elegans P granule condensates. *PNAS,* 2021, vol. 118 (37
- **SAHA et al.** *Cell,* 2016, vol. 166, 1572-1584.e16
- **WANG, J. et al.** A Molecular Grammar Governing the Driving Forces for Phase Separation of Prion-like RNA Binding Proteins. *Cell,* 2018, vol. 174, 688-699.e16
- **FRITSCH et al.** *PNAS,* 2021, vol. 118 (37